# EUROPÄISCHE PATENTSCHRIFT

(11) **EP 2 934 640 B1**
(45) Veröffentlichungstag und Bekanntmachung des Hinweises auf die Patenterteilung: **12.08.2020**
(21) Anmeldenummer: 13815715.1
(22) Anmeldetag: 18.12.2013
(51) Int. Cl.: A61M 16/00, A61M 16/20, A61H 31/00, A61M 16/08, A61M 16/10, A61M 16/12, A61N 1/39

(54) **BEATMUNGSSYSTEM**
VENTILATION SYSTEM
SYSTÈME DE VENTILATION

(30) Priorität: 18.12.2012 DE 102012024672
(43) Veröffentlichungstag der Anmeldung: 28.10.2015
(73) Patentinhaber: Drägerwerk AG & Co. KGaA, 23558 Lübeck (DE)
(72) Erfinder: LÖSER, Judith, 23558 Lübeck (DE); KULLIK, Götz, 23568 Lübeck (DE)
(86) Internationale Anmeldenummer: PCT/EP2013/077035
(87) Internationale Veröffentlichungsnummer: WO 2014/095962

(56) Entgegenhaltungen:
- EP-A1- 2 198 823
- EP-A2- 2 343 097
- WO-A1-2012/131586
- DE-A1-102009 012 146
- US-A- 4 326 507

## Beschreibung

Die vorliegende Erfindung betrifft ein Beatmungssystem mit einer Gasversorgungseinrichtung mit einer Schlauchanordnung, wobei die Schlauchanordnung einen Patientenanschluss zur Verbindung mit einem Patienten aufweist, um Gas von der Gasversorgungseinrichtung zum Patienten zu führen und um vom Patienten ausgeatmetes Gas abzuführen, mit Mitteln zum Steuern des Gasstromes von der Gasversorgungseinrichtung zum Patientenanschluss und zum Steuern des Gasstroms vom Patientenanschluss weg, mit einer Sensoreinheit, die derart in der Schlauchanordnung angeordnet und eingerichtet ist, Parameter des dem Patienten zugeführten und vom Patienten ausgeatmeten Gases zu erfassen, und mit einer Steuer- und Regeleinheit zum Steuern der Gasversorgungseinrichtung und der Mittel zum Steuern des Gasstroms, die mit der Gasversorgungseinrichtung, den Mitteln zum Steuern des Gasstroms und der Sensoreinheit, verbunden ist.

Bei der maschinellen Beatmung von Patienten, insbesondere im Notfalleinsatz ist oftmals eine Situation gegeben, bei der ein Patient sowohl beatmet werden muss, als auch eine Herz- Lungen- Wiederbelebung an diesem Patienten vorgenommen werden muss. Im Regelfall wird der Patient über eine Maske oder einen Endotrachialtubus mit Hilfe eines Notfall- Beatmungsgerätes maschinell beatmet.

Aus dem Stand der Technik sind einerseits Notfallbeatmungsgeräte bekannt, welche die maschinelle Beatmung des Patienten in Notfallsituationen ermöglichen. Notfallbeatmungsgeräte zeichnen sich dadurch aus, dass sie mobil, portabel und autark von externer Versorgung mit elektrischer Energie und Atemgasen einsetzbar sind. Notfallbeatmungsgeräte sind in der DE 4007361 A1, sowie der DE 20315975 U1 gezeigt. Daneben sind portable und mobile Beatmungsgeräte aus dem Bereich der unterstützenden Therapie, sowohl für den klinischen wie auch den häuslichen Einsatz bekannt. So beschreibt die DE 8418594 U1 ein Gerät zur Akutintervention bei Asthma, Herzanfällen, Herzinfarkten, Kreislaufbeschwerden und auch zum langzeitigen Einsatz in der Therapie chronischer Bronchitis. Andererseits sind Geräte bekannt, die dem Anwender eine Hilfestellung bei der Durchführung der Herz- Lungen- Wiederbelebung (HLW) geben. Eine Herz- Lungen- Wiederbelebung (HLW) wird durch eine helfende und rettende Person, ggf. mit Unterstützung einer zweiten Person auf manuelle Weise als ein Wechsel aus einer Druckmassage des Brustkorbs und einer Atemspende mittels Mund- zu- Mund oder Mund- zu- Nase durchgeführt. Der Wechsel zwischen Druckmassage und Atemspende wird üblicherweise in einem fortlaufenden Rhythmus von 30 Brustkorb- Druckmassagen im Wechsel mit 2 Atemspenden, bzw. in einem fortlaufenden Rhythmus von 15 Brustkorb- Massagen im Wechsel mit 2 Atemspenden durchgeführt, bis das Herz- Kreislauf- System des Patienten wieder eigenständig in Funktion ist, das Herz also wieder regelmäßig schlägt. Anschließend wird die Atemspende solange weitergeführt, bis der Patient wieder eigenständig atmet. Man spricht dabei auch von einer 30- zu- 2- Herz- Lungen- Wiederbelebung (HLW), wobei es dazu auch noch weitere Variationen gibt. In der sogenannten Ein- Retter- Methode führt eine Person im Wechsel die Brustkorb- Massagen und die Atemspenden durch, in der sogenannten Zwei- Retter- Methode übernimmt eine Person die die Brustmassage und die zweite Person die Atemspenden. Als eine Hilfestellung für die erste und / oder zweite Person sind gerät verfügbar, welche den 30- zu- 2- Rhythmus durch optische und/ oder akustische Signalgabe unterstützen und es der Person / den Personen ermöglichen, sich selbst im Wesentlichen auf die Durchführung der Herz- Lungen-Wiederbelebung (HLW) und den Zustand des Patienten konzentrieren zu können.

Geräte zur Unterstützung der helfenden und rettenden Personen sind in der US 2006 111 749 A1 beschrieben.

Weiterhin sind Trainingsgeräte und Simulatoren für klinisches Personal zur Einübung der Durchführung der Druckmassage des Brustkorbs mit korrektem Druck im rhythmisch ordnungsgemäßen Wechsel mit der Atemspende mit korrekter Beatmung (Luftmenge) bekannt. Solche Trainingsgeräte und Simulatoren sind in der US 2004 058 305 A1 beschrieben.

Als ein weiterer Stand der Technik ist in der US 8,151,790 ein Ventil genannt, dass für die gleichzeitige Anwendung mit der Herz- Druck- Massage zwischen einem Beatmungsgerät und einem Patienten angeordnet werden kann, um die zeitlichen Füllung der Lunge und den Druck in der Lunge, sowie den zeitlichen Verlauf des Drucks in Bezug zu den vom Beatmungsgerät gelieferten Zeitraster von In- und Exspirationsphasen zu verändern.

Ein kardiopulmonales Gerät zur Wiederbelebung (HLW) eines Patienten zu einer Durchführung einer Herz- Druck- Massage und mit Mitteln zu einer Steuerung eines Beatmungsgerätes ist aus der EP0029352 B1 bekannt. Das Beatmungsgerät wird dabei derart angesteuert, dass synchron zur Herz- Druck- Massage während des Bluttransportes vom Herzen in den Körper des Patienten (Systole) zeitweise eine Ausströmung von Luft aus der Lunge des Patienten unterbunden wird.

Die US 6,155,257 zeigt ein Beatmungsgerät, sowie ein Verfahren zum Betrieb des Beatmungsgerätes in Verbindung mit einer Herz- Lungen- Wiederbelebung (HLW), wobei die Beatmung an die Herz- Lungen- Wiederbelebung (HLW) angepasst ist. Es ist ein Ventil vorgesehen, das im Gasstrom zum Patienten angeordnet ist, um das Einströmen von Gas in die Lunge des Patienten so lange zu verzögern, bzw. zu verhindern, bis der Druck im Thoraxraum des Patienten einen vorbestimmten Unterdruckwert gegenüber dem Umgebungsdruck unterschritten hat.
Aus der DE 10 2009 012 146 A1 ist ein Beatmungsystem nach dem Oberbegriff von Anspruch 1 bekannt. Beschrieben ist ein Verfahren zum Betrieb eines Beatmungsgerätes, mit dem eine Hochfrequenz-Beatmung ausgeführt wird, wobei einem Patienten Beatmungsluft mit einer aufmodulierten Wechselschwingung zuführt wird, ohne dass der mittlere Beatmungsdruck verändert wird. Das Beatmungsgerät weist hierfür ein Inspirations- und ein Exspirationsventil auf, die in einer Atemgaszuführleitung und -auslassleitung angeordnet sind. Diese Leitungen weisen ferner Mittel zur Regelung und Messung des Durchflusses sowie des Drucks auf. Es ist ferner eine Steuer- und Regeleinheit vorgesehen, die einen Stellgrößeneingang umfasst, über den die für die Hochfrequenz-Beatmung relevanten Stellgrößen zur Durchführung der Hochfrequenz-Beatmung in die Steuer- und Regeleinheit eingegeben werden. Die Steuer- und Regeleinheit ist hierfür mit der Durchfluss- und Druckregelung und dem Stellgrößeneingang verbunden.

Die EP 2 343 097 A2 beschreibt eine Vorrichtung und ein Verfahren zur Generierung eines Atemgashubes während einer Herz-Druckmassage. Diese Vorrichtung umfasst eine Sensoreinheit zur Feststellung einer Thoraxkompression, sodass Drucksignale im Bereich des Thorax eines Patienten registriert werden und an eine Gasversorgungseinheit geleitet werden, die einem Patienten Atemgas über eine Atemgaszuführleitung zuführt. Bei Erfassung einer Thoraxkompression wird ein kurzer Druckimpuls (z.B. im Bereich von 205 bis 340 ms) mit erhöhtem Druck erzeugt, der die Kompression des Thorax durch Gegendrücken in der Lunge begleitet.

In einer üblichen Notfallsituation findet bei Einsatz eines Notfallbeatmungsgerätes die Herz- Lungen- Wiederbelebung (HLW) durch einen Helfer zeitgleich und überlagernd, aber im Wesentlichen unabhängig von der Beatmung statt. Dabei ist eine Herz- DruckMassage (HDM) für die Aufrechterhaltung der Kreislauffunktion des Patienten erforderlich, um im Wesentlichen das Gehirn und weitere Körperteile dadurch mit Sauerstoff zu versorgen, dass der Blutfluss vom Herzen über die Lunge in die Körperteile zur Vermeidung Schädigungen, insbesondere von bei Sauerstoffmangel relativ unmittelbar eintretenden bleibenden Schädigungen im Gehirn, aufrechterhalten wird und so eine Zufuhr von Sauerstoff aus der Lunge in die Körperzellen der Körperteile und einen Abtransport von Kohlendioxid aus den Körperzellen sicherzustellen. Daher ist zusätzlich eine Zufuhr von frischer Atemluft mit ausreichendem Sauerstoffanteil zur Lunge erforderlich. Diese Zufuhr kann durch eine manuelle Atemspende eines Helfers mit einer Sauerstoffkonzentration von ungefähr 16% erreicht oder durch die Verwendung eines Notfallbeatmungsgerätes mit variabler und einstellbarerer Sauerstoffzufuhr erfolgen.

Für die Anwendung der Herz- Druck- Massage ist es vorteilhaft, dass das Herz bei einem Eindrücken des Brustkorbes nicht ausweichen kann. Da die Möglichkeiten zum Ausweichen im Wesentlichen durch die Anatomie der Rippen und die Organe direkt unterhalb des Brustraumes (Magen, Milz, Leber) relativ gleichbleibend eingeschränkt sind, verbleibt als Ausdehnungsraum zum Ausweichen der räumliche Bereich der Lunge. Immer dann, wenn die Lunge soweit komplett geleert ist, das nur noch die sogenannte funktionale Residualkapazität (FRC) mit Luft gefüllt ist, also am Ende einer jeden Ausatemphase, der sogenannten Exspirationsphase, ist der räumliche Bereich, in welchen das Herz ausweichen kann, maximal. Wenn für das Herz im Brustraum eine Möglichkeit zum Ausweichen gegeben ist, dann ist der Effekt der Herz- DruckMassage, trotz massiver Kraftaufbringung auf den Brustkorb des Patienten durch den Helfer, in Bezug auf durch die Herz- Druck- Massage bewirkte Förderung des Blutes vom Herzen zu den Körperteilen, insbesondere zum Gehirn, geringer, als wenn dieser Ausweichraum nicht zur Verfügung steht. Als Folge davon ergibt sich ein weniger wirksamer Austausch zwischen dem im Blut vorhandenen Sauerstoff und Kohlendioxid und damit verbunden insbesondere eine weniger gute Versorgung des Gehirns mit Sauerstoff, womit sich für den Patienten die Wahrscheinlichkeit von bleibenden Schädigungen erhöht. Daher ist es bei der Durchführung der Herz- Druck- Massage vorteilhaft, dass sich die Lunge während der Kompressionsphase der Herz- DruckMassage weitgehend gefüllt, bzw. nicht wesentlich entleert ist, so dass der räumliche Bereich, in welchen das Herz ausweichen kann, minimiert wird und damit der Effekt der Herz- Druck- Massage und damit mittelbar auch der Druck des in den Körper strömenden Blutes (systolischer Blutdruck) erhöht wird und damit der Austausch zwischen dem im Blut vorhandenen Sauerstoff und Kohlendioxid verbessert wird.

Weiterhin ist es bei der Durchführung der Herz- Druck- Massage vorteilhaft, dass die Lunge des Patienten in der Dekompressionsphase der Herz- Druck- Massage nahezu vollständig bis auf das Volumen der Funktionellen Residualkapazität entleert wird, idealer Weise sogar ein geringer Unterdruck in der Lunge in Relation zum Umgebungsdruck bewirkt wird, um die Rückströmung des Blutes zum Herzen zu unterstützen. Diese Unterstützung ergibt sich daraus, dass dem Herzen und den zum Herzen führenden venösen Blutgefäßen ausreichend Raum im Brustraum des Patienten zur Rückströmung von Blut zur Verfügung steht und die nahezu entleerte Lunge diesen Raum dann nicht ausfüllt. Ein zusätzlicher dabei Aspekt ist der, dass der Blutdruck des rückströmenden Blutes (diastolischer Blutdruck) nicht durch den in der Lunge herrschenden Beatmungsdruck beeinflusst und dadurch möglicherweise erhöht wird. Als eine Folge der Unterstützung der Rückströmung des Blutes zum Herzen ergibt sich mittelbar über eine Verbesserung der Durchblutung und des Blutaustauschs im Herzen insgesamt damit eine Verbesserung des Austauschs von Sauerstoff und Kohlendioxid im Blutkreislauf. Als eine Folge des verbesserten Austauschs von Sauerstoff und Kohlendioxid im Blut ergibt sich eine Reduzierung der Gefährdung des Patienten, insbesondere des Gehirns des Patienten, hinsichtlich bleibender Schädigungen. Wird eine klassische Herz- Lungen- Wiederbelebung (HLW) nach der ein- Retter- Methode durchgeführt, so kommt der 30- zu- 2- Rhythmus unter Anwendung einer Beatmung durch eine Gesichtsmaske zum Einsatz. Dabei finden Herz- Druck- Massage und Beatmung nicht gleichzeitig statt. Sobald in einer Notfallsituation ein weiterer Retter, insbesondere ein Notarzt zur Verfügung steht, wird die Maske durch einen Endotrachialtubus ersetzt und der Endotrachialtubus mittels einer Schlauchverbindung mit einem Notfall- Beatmungsgerät verbunden wird. Ein solcher Endotrachialtubus wird im Verlauf der weiteren Beschreibung der vorliegenden Anmeldung abkürzend mit "Tubus" bezeichnet. Dies hat den Vorteil, dass der Zugang zur Lunge frei bleibt, da durch den Tubus sichergestellt wird, dass kein vom Patienten aspiriertes Material aus dem Magen- Darm- Bereich bei der Gabe der Atemspende in die Lunge des Patienten transportiert werden kann. Sobald der Zugang zu den Atemwegen des Patienten sichergestellt ist, findet eine kontinuierliche Beatmung durch das Beatmungsgerät statt. Zeitgleich wird von einem Retter oder einer geeigneten Vorrichtung die Herz- Druck- Massage kontinuierlich weitergeführt.

Eine geeignete Vorrichtung zur Applizierung einer maschinellen Herz- Druck- Massage auf dem Brustkorb eines Patienten ist beispielsweise in der EP0509773 B1 beschrieben.

Die kontinuierlichen Kompressionen des Brustraums als Wirkung der Herz- DruckMassage wirken sich, im Unterschied zur Herz- Lungen- Wiederbelebung (HLW) mit Wechsel zwischen Herz- Druck- Massage und Beatmung, beispielsweise nach dem 30 (Herz- Druck- Massagen) zu 2 (Beatmungszyklen) Rhythmus 15 zu 2 Rhythmus oder 10 zu 2 Rhythmus, sowohl auf die Art und Weise der Füllung der Lunge durch das Beatmungsgerät, wie auch auf den resultierenden Druckverlauf in der Lunge aus.

Es findet somit im zeitlichen Verlauf des messtechnisch erfassten Beatmungsdrucks im Betrieb des Beatmungsgerätes eine Überlagerung der durch die Beatmung und die gewählte Beatmungsform bedingten Druckwechsel aus Exspiration und Inspiration und der Kompressionen der Herz- Druck- Massage statt. Zur Beatmung eines Patienten werden von einem Beatmungsgerät nach dem Stand der Technik drei prinzipielle Basis- Beatmungsformen bereitgestellt, Varianten von Druck- geregelten Beatmungsformen, Varianten von Volumen- geregelten Beatmungsformen, Varianten von Durchfluss (Flow-) - geregelten Beatmungsformen, sowie Kombinationen davon, wie beispielsweise eine Druck- geregelten Beatmungsform mit Volumengarantie und Maximal- Flow- Limitierung. Diese Überlagerung durch die und der Kompressionen der Herz- Druck- Massage stellt eine zusätzliche Randbedingung und eine Störgröße für die Regelung des Beatmungsdrucks, insbesondere bei den Druck- geregelten Beatmungsformen dar.

Es ist die Aufgabe der vorliegenden Erfindung, ein Beatmungssystem zu einer Unterstützung einer Herz- Lungen- Wiederbelebung (HLW), sowie ein geeignetes Verfahren zu einer Unterstützung einer Herz- Lungen- Wiederbelebung (HLW) anzugeben.

Diese Aufgabe wird gelöst durch ein Beatmungssystem mit den Merkmalen des Anspruchs 1.

Bevorzugte Ausführungsformen des Beatmungssystems sind in den Unteransprüchen aufgeführt.

Das erfindungsgemäße Beatmungssystem kann somit in einer ersten Betriebsart arbeiten, bei der der Verlauf des Drucks des Atemgases, mit dem der Patient versorgt wird, während der Inspirations- als auch der Exspirationsphase einen ersten bzw. einen zweiten Verlauf hat. Das bedeutet, dass der Druck während des ersten bzw. zweiten Verlaufs zeitlich aufeinanderfolgend eine Reihe von Werten annimmt, die in der Steuer- und Regeleinheit hinterlegt sind. Diese Verläufe sind für eine normale Beatmung eines Patienten vorgesehen und können in Abhängigkeit vom Patienten variieren.

Darüber hinaus ist das erfindungsgemäße Beatmungssystem und dessen Steuer- und Regeleinheit jedoch so ausgestaltet, dass eine zweite Betriebsart vorgesehen ist, in der sowohl in der Inspirationsphase als auch in der Exspirationsphase für den Druck, den das Atemgas hat, das dem Patienten während dieser Phasen zugeführt wird, jeweils in einer ersten Teilphase der Exspirationsphase bzw. Inspirationsphase von dem ersten bzw. zweiten Verlauf abweichende Verläufe gewählt werden.

Diese zweiten und dritten Verläufe sind so gewählt, dass in der ersten Teilphase der Exspirationsphase der Verlauf des Drucks, also der dritte Verlauf, gegenüber dem Verlauf in der ersten Betriebsart erhöht ist. Dies bedeutet, dass in der Exspirationsphase der zweiten Betriebsart die sich dann für den Druck des Atemgases ergebenden Werte in der ersten Teilphase über denen liegen, die sich zu entsprechenden Zeitpunkten in der ersten Betriebsart ergeben.

Wenn die zweite Betriebsart dann gewählt wird, wenn an dem Patient eine Herz-Lungen-Wiederbelebung vorgenommen wird, ist in der ersten Teilphase der Exspirationsphase der Druck in der Lunge gegenüber der normalen Beatmung in der ersten Betriebsart erhöht. Dies hat zur Folge, dass bei der mit der Herz-Lungen-Wiederbelebung verbundenen Kompression des Brustkorbes das Herz weniger stark in den Bereich der Lunge ausweichen kann und damit stärker komprimiert wird, so dass das Blut in größerem Umfang aus dem Bereich des Herzens heraus gedrückt wird.

Darüber hinaus wird in der zweiten Betriebsart in der ersten Teilphase der Inspirationsphase der Verlauf des Drucks, also definitionsgemäß der vierte Verlauf, derart gewählt, dass er gegenüber dem Verlauf in der ersten Betriebsart, also dem zweiten Verlauf, zumindest abschnittsweise niedrigere Werte annimmt. Dies bedeutet auch hier, dass die sich ergebenden Werte für den Druck des Atemgases am Patientenanschluss in der ersten Teilphase der Inspirationsphase unten denen liegen, die sich zu entsprechenden Zeitpunkten der Inspirationsphase in der ersten Betriebsart ergeben. Dieser vierte Verlauf ist also gegenüber dem zweiten Verlauf abgesenkt. Dies führt dann, wenn die zweite Betriebsart zusammen mit einer Herz-Lungen-Wiederbelebung aktiviert wird, dazu, dass sich die Lunge des Patienten anfänglich stärker zusammenzieht und sich das Herz in größerem Umfang ausdehnen kann und Blut somit in größerem Umfang zurück in die Blutgefäße des Herzens zurückströmt, als dies der Fall wäre, wenn der Patient gemäß der ersten Betriebsart beatmet würde.

Durch das Vorsehen von zwei Betriebsarten bei dem erfindungsgemäßen Beatmungssystem wird also erreicht, dass dessen Betrieb daran angepasst werden kann, ob der Patient eine Herz-Lungen-Wiederbelebung erfährt oder nicht und dadurch die Wirkung der Wiederbelebungsmaßnahme optimiert wird.

Die Steuer- und Regeleinheit ist dazu ausgestaltet, die Mittel zum Steuern des Gastroms derart anzusteuern, dass in der zweiten Betriebsart die Exspirationsphase und die Inspirationsphase zwei aufeinanderfolgende Teilphasen aufweisen, wobei in der ersten Teilphase der Exspirationsphase der Druck am Patientenanschluss des vom Patienten ausgeatmeten Gases den dritten Verlauf hat, wobei in der zweiten Teilphase der Exspirationsphase der Druck am Patientenanschluss des vom Patienten ausgeatmeten Gases einen dem ersten Verlauf entsprechenden Verlauf hat, wobei in der ersten Teilphase der Inspirationsphase der Druck am Patientenanschluss des dem Patienten zugeführten Gases den vierten Verlauf hat und wobei in der zweiten Teilphase der Inspirationsphase der Druck am Patientenanschluss des dem Patienten zugeführten Gases einen dem zweiten Verlauf entsprechenden Verlauf hat.

Durch diese Ausgestaltung des Beatmungssystems wird die Beatmung des Patienten während der Anwendung einer Herz-Lungen-Wiederbelebung derart verändert, dass in der dann ausgelösten zweiten Betriebsart der Druck des Atemgases am Patientenanschluss in der Inspirationsphase nur an deren Anfang, nämlich in der ersten Teilphase, erhöht wird, während er in dem weiteren Teil der Inspirationsphase, nämlich der zweiten Teilphase, den gleichen Verlauf hat wie im Normalbetrieb, also in der ersten Betriebsart. In analoger Weise wird die Exspirationsphase auch in zwei Teilphasen unterteilt, wobei hier nur in der zeitlich gesehen ersten Teilphase eine Erniedrigung des Drucks gegenüber der ersten Betriebsart stattfindet, während in der zweiten Teilphase der Verlauf des Drucks dem entspricht, wie dies auch während der normalen Beatmung der Fall ist, also in der ersten Betriebsart.

Dadurch, dass es nur am Anfang der Inspirations- und Exspirationsphase zu Veränderungen des Verlaufs des Drucks kommt, wird einerseits der gewünschte Effekt der verstärkten Blutzirkulation am Herzen erreicht, die Atmung des Patienten aber nicht über die gesamte Espirations- und Inspirationsphase erschwert.

Um die Druckerhöhung am Beginn der Exspirationsphase sowie die Verringerung am Beginn der Inspirationsphase zu erreichen, ist es in bevorzugter Weise möglich, dass die Steuer- und Regeleinheit ausgestaltet ist, während der Exspirationsphase die Mittel zum Steuern des Gastroms derart anzusteuern, dass der dritte Verlauf des Drucks durch Erhöhung eines Drucksollwerts erreicht wird. Außerdem kann die Steuer- und Regeleinheit ausgestaltet sein, während der Inspirationsphase die Mittel zum Steuern des Gastroms derart anzusteuern, dass der vierte Verlauf des Drucks durch Verringerung eines Drucksollwerts erreicht wird.

Als Alternative dazu kann die Schlauchanordnung eine Atemgasauslassleitung aufweisen, die vom Patientenanschluss wegführt und gegenüber dem Patientenanschluss durch ein Exspirationsventil geöffnet und verschlossen werden kann, wobei das Exspirationsventil mit der Steuer- und Regeleinheit verbunden ist und wobei die Steuer- und Regeleinheit ausgestaltet ist, während der Exspirationsphase das Exspirationsventil derart anzusteuern, dass es gegenüber dem Beginn der Exspirationsphase zeitlich verzögert geöffnet wird. In gleicher Weise kann die Schlauchanordnung eine Atemgaszuführleitung aufweisen, die von der Gasversorgungseinrichtung zum Patientenanschluss führt und gegenüber dem Patientenanschluss durch ein Inspirationsventil geöffnet und verschlossen werden kann, wobei das Inspirationsventil mit der Steuer- und Regeleinheit verbunden ist, wobei die Steuer- und Regeleinheit ausgestaltet ist, während der Inspirationsphase das Inspirationsventil derart anzusteuern, dass es gegenüber dem Beginn der Inspirationsphase zeitlich verzögert geöffnet wird.

Bei dieser Alternative wird die Druckerhöhung am Beginn der Exspirationsphase bzw. die Erniedrigung am Beginn der Inspirationsphase dadurch in vergleichsweise einfacher Weise erreicht, dass die entsprechenden Ventile in der Schlauchanordnung verzögert geöffnet werden, sodass die Atmung des Patienten und der fehlende Abfluss bzw. Zustrom von Gas zu der Druckänderung im Vergleich zum ersten bzw. zweiten Verlauf führt.

In bevorzugter Weise ist eine Umschalteinrichtung am Beatmungssystem vorgesehen, mit der ein Benutzer die Steuer- und Regeleinheit zwischen der ersten und zweiten Betriebsart umschalten kann. Dabei kann die Umschalteinrichtung auch über Software realisiert werden, über die das System gesteuert wird und die dann eine Funktion bereitstellt, durch die ein Benutzer auf einer Benutzeroberfläche zwischen den Betriebsarten hin und herschalten kann.

Die automatische Erfassung einer Herz-Lungen-Wiederbelebung kann in weiter bevorzugter Weise dadurch erfolgen, dass die Steuer- und Regeleinheit ausgestaltet ist, den zeitlichen Verlauf des Drucks am Patientenanschluss während der Inspirationsphase und der Exspirationsphase zu überwachen und aus dem zeitlichen Verlauf des Drucks zu bestimmen, ob an dem Patienten eine Herz-Lungen-Wiederbelebung ausgeführt wird. Hierbei kann insbesondere das Drucksignal auf regelmäßig auftretende Spitzen überwacht werden und dies als Kriterium dafür verwendet werden, dass eine Herz-Lungen-Wiederbelebung durchgeführt wird.

In einer weiteren bevorzugten Ausführungsform weist das Beatmungssystem eine Anzeigeeinheit auf, die mit der Steuer- und Regeleinheit verbunden ist, wobei die Steuer- und Regeleinheit ausgestaltet ist, dass in der ersten Betriebsart bei Überschreiten oder Unterschreiten eines Schwellwerts durch einen von der Sensoreinheit erfassten Parameter eine erste Alarmmeldung auf der Anzeigeeinrichtung ausgegeben wird, und wobei die Steuer- und Regeleinheit ausgestaltet ist, in der zweiten Betriebsart bei Überschreiten oder Unterschreiten des Schwellwerts durch den von der Sensoreinheit erfassten Parameter keine Alarmmeldung oder eine sich von der ersten Alarmmeldung unterscheidende zweite Alarmmeldung auszugeben.

Durch diese Ausgestaltung wird berücksichtigt, dass die Herz-Lungen-Wiederbelebung die von der Sensoreinheit erfassten Parameter wie beispielsweise den Druck oder den CO₂-Gehalt beeinflusst. Alarmeinstellungen, die in der ersten Betriebsart, bei der der Patient normal beatmet wird, sinnvoll sind, haben in der zweiten Betriebsart während einer Herz-Lungen-Wiederbelebung keinen Sinn mehr, so dass ein von der Anzeigeeinheit ausgegebener Alarm nicht mehr sinnvoll ist, sondern den Anwender eher stört. Diesem trägt diese Ausführungsform Rechnung.

Wenn die Anzeigeeinheit akustische Signalmittel zum Ausgeben eines akustischen Alarms aufweist, wobei die erste Alarmmeldung einen ersten akustischen Alarm umfasst, kann die zweite Alarmmeldung keinen akustischen Alarm oder einen sich von dem ersten unterscheidenden zweiten akustischen Alarm umfassen, dessen Lautstärke gegenüber dem ersten akustischen Alarm verringert ist.

In anderen bevorzugten Ausführungsform oder in Kombination mit der zuvor erläuterten ist eine Anzeigeeinheit vorgesehen, wobei die Steuer- und Regeleinheit ausgestaltet ist, in der zweiten Betriebsart einen von der Sensoreinheit erfassten Parameter zu überwachen und bei Unterschreiten eines ersten Schwellwerts durch den Parameter eine erste Meldung durch die Anzeigeeinheit auszulösen, bei Überschreiten des ersten Schwellwerts und Unterschreiten eines zweiten Schwellwerts, der größer als der erste Schwellwert ist, durch den Parameter eine zweite Meldung durch die Anzeigeeinheit auszulösen und bei Überschreiten des zweiten Schwellwerts durch den Parameter eine dritte Meldung durch die Anzeigeeinheit auszulösen, und wobei sich die erste, zweite und dritte Meldung voneinander unterscheiden. Insbesondere kann dabei die Sensoreinheit einen Sensor aufweisen, der ausgestaltet ist, während der Exspirationsphase den CO₂-Gehalt der von dem Patienten ausgeatmeten Luft zu bestimmen, wobei der CO₂-Gehalt der vom Patienten ausgeatmeten Luft der überwachte Parameter ist.

Bei einer solchen Ausgestaltung wird der Anwender durch die unterschiedlichen Meldungen informiert, ob der fragliche Parameter in einem ggf. gewünschten Bereich zwischen dem ersten und zweiten Schwellwert liegt oder außerhalb davon. Im Fall des CO₂-Gehalts in der ausgeatmeten Luft kann der erste Schwellwert so gewählt werden, dass ein gemessener Wert unterhalb davon darauf hinweist, dass die Herz-Lungen-Wiederbelebung keine hinreichende Wirkung zeigt und möglicherweise nicht richtig ausgeführt wird. Der zweite Schwellwert kann so gewählt werden, dass dann, wenn der gemessene CO₂-Gehalt darüber liegt, die Herz-Lungen-Wiederbelebung abgebrochen werden kann, weil der Patient eigenständig atmen kann. Dann zeigt die zweite Meldung, dass die Herz-Lungen-Wiederbelebung korrekt ausgeführt wird aber fortgesetzt werden muss, während die dritte Meldung dem Anwender zeigt, dass die Herz-Lungen-Wiederbelebung beendet werden kann.

Alternativ zu einem Parameter der Atemluft kann zu dem zuvor erwähnten Zweck am Beatmungssystem ein Sensor zur Messung der Sauerstoffsättigung im Blut (SPO₂) vorgesehen sein, der mit einem Patienten verbunden werden kann, wobei die Steuer- und Regeleinheit ausgestaltet ist, in der zweiten Betriebsart die Sauerstoffsättigung im Blut (SPO₂) zu überwachen und bei Unterschreiten eines ersten Schwellwerts durch die Sauerstoffsättigung im Blut (SPO₂) eine erste Meldung durch die Anzeigeeinheit auszulösen, bei Überschreiten des ersten Schwellwerts und Unterschreiten eines zweiten Schwellwerts, der größer als der erste Schwellwert ist, durch die Sauerstoffsättigung im Blut (SPO₂) eine zweite Meldung durch die Anzeigeeinheit auszulösen und bei Überschreiten des zweiten Schwellwerts durch die Sauerstoffsättigung im Blut (SPO₂) eine dritte Meldung durch die Anzeigeeinheit auszulösen, und wobei sich die erste, zweite und dritte Meldung voneinander unterscheiden.

Schließlich kann das Beatmungssystem eine Einrichtung zur selbsttätigen Durchführung einer Herz-Lungen-Wiederbelebung aufweisen, die mit der Steuer- und Regeleinheit verbunden ist, wobei die Steuer- und Regeleinheit ausgestaltet ist, bei einer Aktivierung der Einrichtung zur Durchführung einer Herz-Lungen-Wiederbelebung von der ersten in die zweite Betriebsart umzuschalten.

Außerdem ist es möglich, dass das Beatmungssystem einen Spannungsgenerator zum Erzeugen von Spannungspulsen aufweist, der mit der Kontrolleinheit verbunden ist, wobei der Spannungsgenerator mit Elektroden zur Verbindung mit einem Patienten versehen ist.

Einige Ausführungsbeispiele der Erfindung sind in den Figuren gezeigt und sind im Folgenden näher erläutert. Es zeigen:
- Figur 1a: eine schematische Übersicht eines Beatmungsgerätes,
- Figur 1b: eine schematische Übersicht eines Notfall- Beatmungsgerätes,
- Figur 2: eine schematische Darstellung einer Beatmungssteuerung nach den Figur 1 a und 1b,
- Figur 3: eine Darstellung von Konzentrationsbereichen der CO₂-Konzentration,
- Figur 4: einen schematischer Ablauf des Verfahrens zum Betrieb eines Beatmungsgerätes mit einer Unterstützung einer Herz- Lungen- Wiederbelebung (HLW),
- Figuren 5a bis 5e: Darstellungen eines zeitlichen Verlaufs von Beatmungsdruck, Herz- Druck- Druckmassage (HDM), CO₂- Messung,
- Figur 6: eine Darstellung zum Zeitverlauf einer Beatmung bei Betrieb eines Beatmungsgerätes mit und ohne einer Unterstützung einer Herz- Lungen- Wiederbelebung (HLW),
- Figur 7a: eine schematische Übersicht eines Medizinischen Systems mit einem Beatmungsgerät nach Figur 1 a oder Figur 1b und mit einem Unterstützungsgerät zur automatischen Durchführung einer Herzdruckmassage (HDM),
- Figur 7b: eine schematische Übersicht eines Medizinischen Systems mit einem Beatmungsgerät nach Figur 1 a oder Figur 1b und mit einem zu einer Reanimation geeigneten Spannungsgenerator.

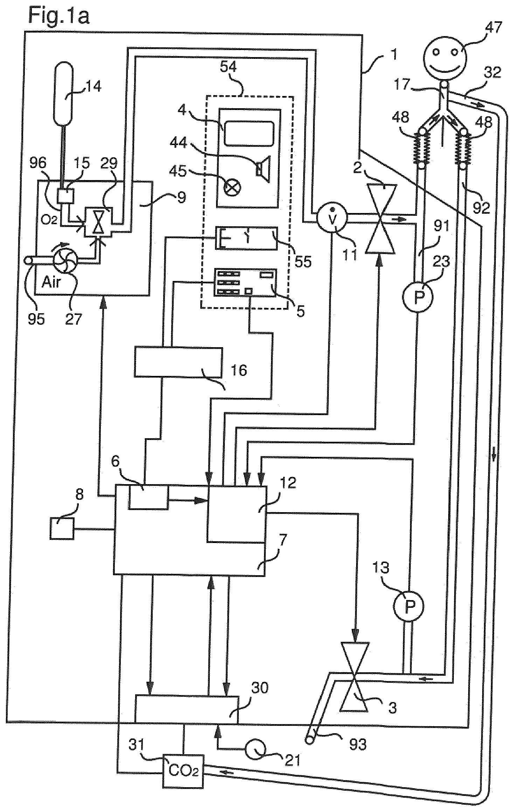

In der Figur 1a ist eine erste schematische Übersicht der Komponenten eines Beatmungsgerätes 1 dargestellt, welches für die Ausführung einer Beatmung ausgerüstet ist. Das Beatmungsgerät 1 weist die folgenden Komponenten auf:
Einem Inspirationsventil 2, einem Exspirationsventil 3, einer Anzeige- und Signalgabeeinheit 4, einer Eingabeeinheit 5, einer Steuer- und Regeleinheit 7, einer Spannungsversorgungseinheit 8, einer Gasmischungs- und Dosiereinheit 9 mit einem als eine Gebläseeinheit 27 ausgebildeten Beatmungsantrieb und mit einem Sauerstoff-Luft- Misch und Dosierventil 29, einer Luft- Gaszuführung 95 und einer Sauerstoff-Gaszuführung 96, einer Durchflussmessung 11, einer Druck- und Durchflussregelung 12, einer exspiratorischen Druckmessung 13, einer inspiratorischen Druckmessung 23, einer Sauerstoffdruckflasche 14 mit einer Druckreduziereinheit 15, einem inspiratorischen Gasanschluss 91, einem exspiratorischen Gasanschluss 92 und einem Gasauslass 93. Weiterhin ist ein Stellgrößeneingang 6 vorhanden, mittels dessen für die Beatmung relevante Einstellgrößen 16, wie Beatmungsfrequenz [RR], Druckamplitude [P_{amplitude}], mittlerer positiver Zieldruck [P] der Beatmung, Tidalvolumen [V_{T}], I:E- Verhältnis [Ratio_{I:E}] von der Eingabeeinheit 5 an die Steuer- und Regeleinheit 7 gelangen und von dort zur Druck- und Durchflussregelung 12 weitergegeben werden. Diese Einstellgrößen 16 dienen als Sollvorgaben für den Beginn und für die Durchführung der Beatmung. Der Patient 47 ist mittels eines Verbindungsstücks (Y-Stück) 17 über einen inspiratorischen Gasanschluss 91 und einen exspiratorischen Gasanschluss 92 mit Hilfe von zwei Zuleitungen 48, in diesem dargestellten Fall nach Figur 1a über ein Zwei- Schlauchsystem, mit dem Beatmungsgerät 1 verbunden. Die Exspirationsluft entweicht über den Gasauslass 93 aus dem Beatmungsgerät 1 in die Umgebung. In der Anzeige- und Signalgabeeinheit 4 sind ein akustisches Signalmittel 44, beispielsweise in Form einer Hupe oder eines Lautsprechers, sowie ein optisches Signalmittel 45, beispielsweise als eine Lampe, eine LED oder ein anderes optisches Anzeigeelement ausgeführt, enthalten. Die Eingabeeinheit 5 kann mit der Anzeige- und Signalgabeeinheit 4 in einer Benutzerschnittstelle 54 kombiniert ausgebildet sein, wobei zusätzlich ein oder mehrere Bedienelemente 55, beispielsweise als Tast- oder Schaltelemente oder als eine Tastatur ausgebildet, mit integriert sein können. Die Bedienelemente 55 sind in der vorliegenden Erfindung dazu ausgebildet, am Beatmungsgerät 1 das Verfahren mit Unterstützung der Herz- Lungen-Wiederbelebung (HLW) zu starten, in Zusammenwirkung mit der Steuer- und Regeleinheit 7 zu steuern und zu konfigurieren oder zu beenden. Weiterhin ist eine Daten- Schnittstelle 30 am Beatmungsgerät 1 vorgesehen. Über diese DatenSchnittstelle 30 kann zusätzliche Sensorik oder Zubehör direkt mit einem unidirektionalen oder einem bidirektionalen Datenaustausch an das Beatmungsgerät 1 angeschlossen werden, oder es kann ein unidirektionaler oder bidirektionaler Austausch von Daten 21 vom Beatmungsgerät 1 mit externen Geräten vorgenommen werden. In dieser Figur 1a ist als externer physiologischer Sensor ein "CO₂-Sidestream- Sensor" 31 dargestellt, der mittels einer Absaugeleitung 32 vom Verbindungsstück (Y- Stück) 17 Atemluft absaugt und hinsichtlich der KohlendioxidKonzentration analysiert und mit der Daten- Schnittstelle 30 verbunden ist.

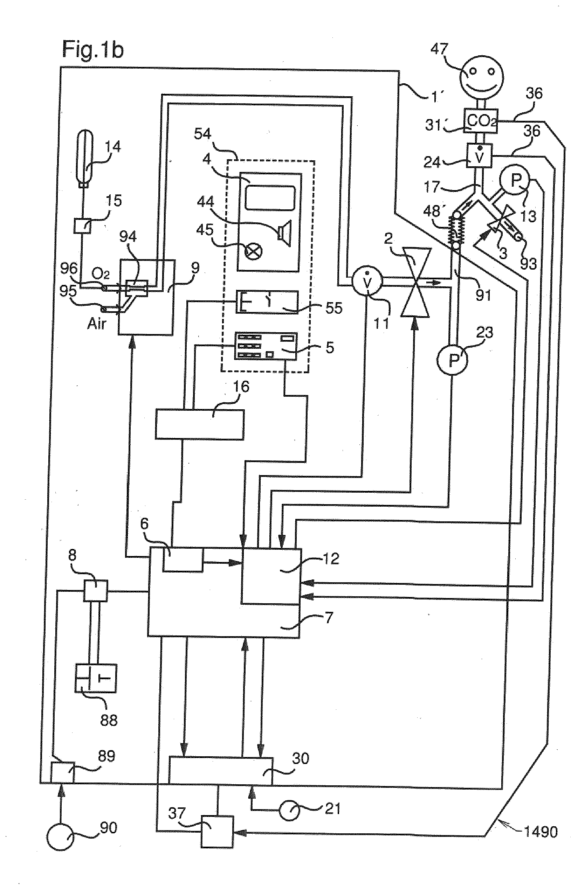

In der Figur 1b ist eine zweite schematische Übersicht der Komponenten eines NotfallBeatmungsgerätes 1' dargestellt, welches für die Ausführung einer Notfall-Beatmung ausgerüstet ist. Gleiche Komponenten in den Figuren 1a und 1b sind mit denselben Bezugsziffern bezeichnet. Das Notfall- Beatmungsgerät 1' weist die folgenden Komponenten auf:
Einem Inspirationsventil 2 einem Exspirationsventil 3, einer Anzeige- und Signalgabeeinheit 4, einer Eingabeeinheit 5, einer Steuer- und Regeleinheit 7, einer Spannungsversorgungseinheit 8 einer Gasmischungs- und Dosiereinheit 9 mit einem Ejektor 94, einer Luft- Gaszuführung 95 und einer Sauerstoff- Gaszuführung 96, einer Durchflussmessung 11, einer Druck- und Durchflussregelung 12, einer exspiratorischen Druckmessung 13', einer inspiratorischen Druckmessung 23 einer Sauerstoffdruckflasche 14 mit einer Druckreduziereinheit 15 und einem inspiratorischen Gasanschluss 91. Weiterhin ist ein Stellgrößeneingang 6 vorhanden, mittels dessen für die Beatmung relevante Einstellgrößen 16, wie Beatmungsfrequenz [RR], Druckamplitude [P_{amplitude}], mittlerer positiver Zieldruck [P] der Beatmung, Tidalvolumen [V_{T}], I:E- Verhältnis [Ratio_{I:E}] von der Eingabeeinheit 5 an die Steuer- und Regeleinheit 7 gelangen und von dort zur Druck- und Durchflussregelung 12 weitergegeben werden. Diese Einstellgrößen 16 dienen als Sollvorgaben für den Beginn und für die Durchführung der Notfall-Beatmung. Der Patient 47 ist mittels eines Verbindungsstücks (Y- Stück) 17 über einen inspiratorischen Gasanschluss 91 mit Hilfe einer Zuleitung 48', in diesem dargestellten Fall nach dieser Figur 1b über ein Ein-Schlauchsystem, mit dem Notfall-Beatmungsgerät 1 verbunden. Die Exspirationsluft entweicht über einen Gasauslass 93' direkt am Verbindungssstück 17 in die Umgebung. In der Anzeige- und Signalgabeeinheit 4 sind ein akustisches Signalmittel 44, beispielsweise in Form einer Hupe oder eines Lautsprechers, sowie ein optisches Signalmittel 45, beispielsweise als eine Lampe, eine LED oder ein anderes optisches Anzeigeelement ausgeführt, enthalten. Die Eingabeeinheit 5 kann mit der Anzeige- und Signalgabeeinheit 4 in einer Benutzerschnittstelle 54 kombiniert ausgebildet sein, wobei zusätzlich ein oder mehrere Bedienelemente 55, beispielsweise als Tast- oder Schaltelemente oder als eine Tastatur ausgebildet, mit integriert sein können. Die Bedienelemente 55 sind in der vorliegenden Erfindung dazu ausgebildet, am Notfall-Beatmungsgerät 1' das Verfahren mit Unterstützung der Herz- Lungen-Wiederbelebung (HLW) zu starten, in Zusammenwirkung mit der Steuer- und Regeleinheit 7 zu steuern und zu konfigurieren oder zu beenden. Weiterhin ist eine Schnittstelle für elektrische Energie 90 vorgesehen. An die Spannungsversorgungseinheit 8 ist ein wieder- aufladbares Batteriepack 88 angeschlossen, welches mittels eines Energielade- und Versorgungselementes 89 und über die Schnittstelle für elektrische Energie 90 von extern mit elektrischer Energie versorgt und geladen werden kann. Weiterhin ist eine Daten- Schnittstelle 30 am Notfall- Beatmungsgerät 1' vorgesehen. Über diese Daten- Schnittstelle 30 können zusätzliche Sensoren oder weitere Geräte direkt mit einem unidirektionalen oder einem bidirektionalen Datenaustausch an das Notfall- Beatmungsgerät 1' angeschlossen werden oder es können Daten 21 externer Geräte mit dem Notfall- Beatmungsgerät 1' unidirektional oder bidirektional über die Daten- Schnittstelle 30 ausgetauscht werden.

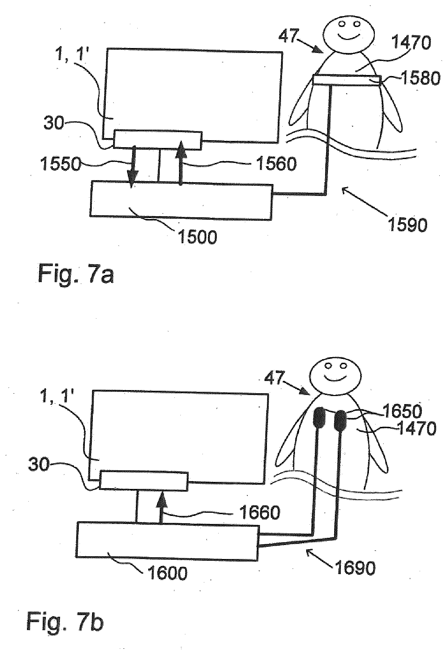

In dieser Figur 1b sind als zusätzliche Sensoren ein Physiologischer Sensor 31' als ein "CO₂- Mainstream- Sensor" zur Bestimmung einer Kohlendioxid- Konzentration (CO₂) im Atemgas des Patienten 47 und ein Patientennaher Durchflusssensor 24 zur Ermittlung der Durchflussmengen zum und vom Patienten 43 am Verbindungsstück (Y-Stück) 17 an das Notfall- Beatmungsgerät 1' angeschlossen. Der Physiologischer Sensor 31' und der Patientennaher Durchflusssensor 24 übermitteln mittels Datenleitungen 36 Messwerte an ein zugehöriges CO₂- Analysegerät 37. Das zugehörige Analysegerät 37 übermittelt über die Datenschnittstelle 30 die Messwerte weiter an das Notfall- Beatmungsgerät 1'. Die Anordnung des "CO₂- Mainstream-Sensors" 31' und des Patientennahen Durchflusssensor 24 am Verbindungsstück in räumlicher Nähe zueinander ist insbesondere vorteilhaft zu einer Bildung eines aus den Messwerten dieser beiden Sensoren abgeleiteten Größe, eines "Minuten-Volumen an Kohlendioxid", MVCO₂. Dieses Minuten- Volumen- CO₂ (MVCO₂) kann in vorteilhafter Weise zu einer Beurteilung einer Qualität einer durchgeführten Herz-Druck- Massage (HDM) in der Vorrichtung zu einer Unterstützung einer Herz- Lungen-Wiederbelebung (HLW), sowie in dem Verfahren zu einer Unterstützung einer Herz-Lungen- Wiederbelebung (HLW) genutzt werden. Über die Datenschnittstelle 30 sind, wie in den Figuren 7a und 7b näher gezeigt, an das Beatmungsgerät 1 (Figur 1a) oder das Notfallbeatmungsgerät 1' (Figur 1b) weitere externe Geräte, wie beispielsweise ein Spannungsgenerator zur Reanimation der Herzkreislauffunktion (Defibrillator) oder ein Unterstützungs- Gerät zu einer automatischen Durchführung einer Herzdruckmassage (HDM) anschließbar, so dass einerseits Informationen und / oder Daten vom externen Gerät, wie beispielsweise EKG- Signale des Defibrillators, anderseits auch Informationen vom Beatmungsgerät 1, 1' an das externe Gerät zu dessen Steuerung, beispielsweise Start- / Stop- / Pause- Signale ausgetauscht werden können, um die Vorrichtung zu einer Unterstützung einer Herz- Lungen-Wiederbelebung (HLW) zu unterstützen, sowie in das Verfahren zu einer Unterstützung einer Herz- Lungen- Wiederbelebung (HLW) mit einzubeziehen.

Die Figur 1b zeigt in schematischer Weise mit dem Beatmungsgerät 1' in Kombination mit dem über die Sensor- und Daten- Schnittstelle 30 angebundenen CO₂-Analysegerät 37 und dem mittels Datenleitung 36 angebundenen Kohlendioxid Sensor 31' als Physiologischem Sensor eine Variante eines Medizinischen Systems 1490.

Ein weiteres und im Wesentlichen ähnliches Medizinisches System ergibt sich gemäß der Figur 1a mit dem Beatmungsgerät 1 in Kombination mit dem über die Sensor- und Daten- Schnittstelle 30 angebundenen Physiologischen Sensor 31.

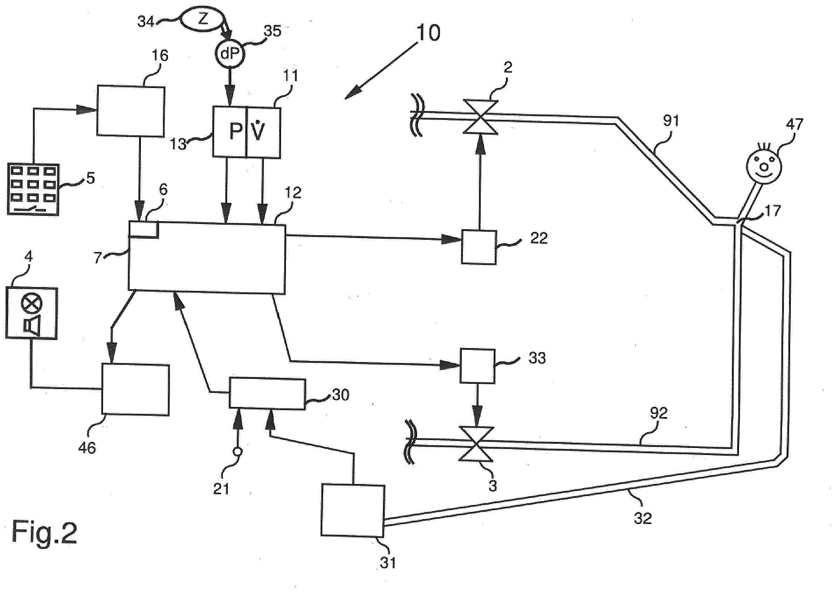

In der Figur 2 sind beispielhaft in einer Darstellung 10 für eine Durchführung des Verfahrens zum Betrieb eines Beatmungsgerätes mit einer Unterstützung einer Herz-Lungen- Wiederbelebung (HLW) wesentliche Elemente des Beatmungsgerätes 1, 1" nach den Figur 1a, 1b gezeigt. Gleiche Elemente in den Figuren 1a, 1b und 2 sind mit den gleichen Bezugsziffern wie in den Figuren 1a, 1b bezeichnet. Gezeigt sind entsprechend den Figuren 1a, 1b das Inspirationsventil 1, das Exspirationsventil 3, die Anzeige- und Signalgabeeinheit 4, die Eingabeeinheit 5, die Druckmessung 13, die Durchflussmessung 11, die Steuer- und Regeleinheit 7 mit der Druck- und Durchflussregelung 12 und dem Stellgrößeneingang 6 für die Eingangsgrößen 16, die Daten- Schnittstelle 30 für Daten 21, das als CO₂- Sensor ausgebildetes Gerät 31, das über die Absaugeleitung 32 und das Verbindungsstück 17 in einer Luft- führender Verbindung mit dem Patienten 47 steht. Der Patient ist über den inspiratorischen Gasanschluss 91 und den exspiratorischen Gasanschluss 93 und das Verbindungsstück mit dem Beatmungsgerät 1, 1' (Figur 1a, 1b) verbunden. In dieser Darstellung 10 in Figur 2 sind folgende Elemente zur Veranschaulichung im Detail dargestellt. Es ist dem Inspirationsventil 2 in dieser Darstellung 10 symbolisch ein Anpassungs- und Verzögerungselement 22 vorgeschaltet. Dem Inspirationsventil 3 ist in dieser Darstellung 10 ebenso symbolisch ein Anpassungs- und Verzögerungselement 33 vorgeschaltet. Die Anpassungs- und Verzögerungselemente 22, 33 sind in einer technischen Umsetzung bevorzugt als Bestandteile der Steuer- und Regeleinheit 7 ausgeführt. Das Inspiratorische Verzögerungselement 22 und das Exspiratorische Verzögerungselement 33 werden durch die Steuerungs- und Regeleinheit 7 angesteuert und im Betrieb des Beatmungsgerätes 1, 1' (Figur 1a, 1b) mit Unterstützung der Herz- Lungen- Wiederbelebung (HLW) in einer Weise aktiviert, so dass die Regelung der Beatmung nicht unmittelbar auf jegliche Änderungen im gemessenen Beatmungsdruck reagiert. Weiterhin wird das Exspiratorische Verzögerungselement 33 von der Steuerungs- und Regeleinheit 7 so angesteuert, dass im Betrieb des Beatmungsgerätes mit Unterstützung der Herz- Lungen-Wiederbelebung (HLW) der Beginn der Exspirationsphase verzögert wird. Weiterhin wird das Inspiratorische Verzögerungselement 22 von der Steuerungs- und Regeleinheit 7 so angesteuert, dass im Betrieb des Beatmungsgerätes mit Unterstützung der Herz- Lungen- Wiederbelebung (HLW) der Beginn der Inspirationsphase verzögert wird. Weiterhin ist in dieser Darstellung 10 gezeigt, dass zwischen der Steuer- und Regeleinheit 7 und der Anzeige- und Signalgabeeinheit 4 ein Alarmierungs- und Alarmanpassungseinheit 46 zwischengeschaltet ist.

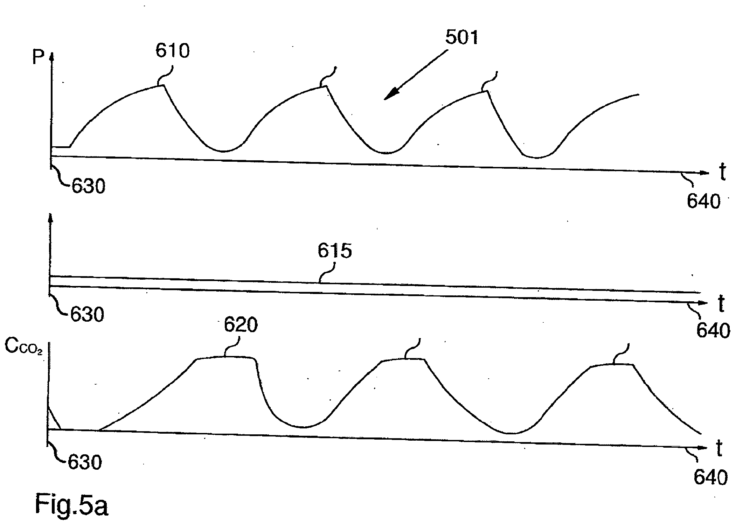

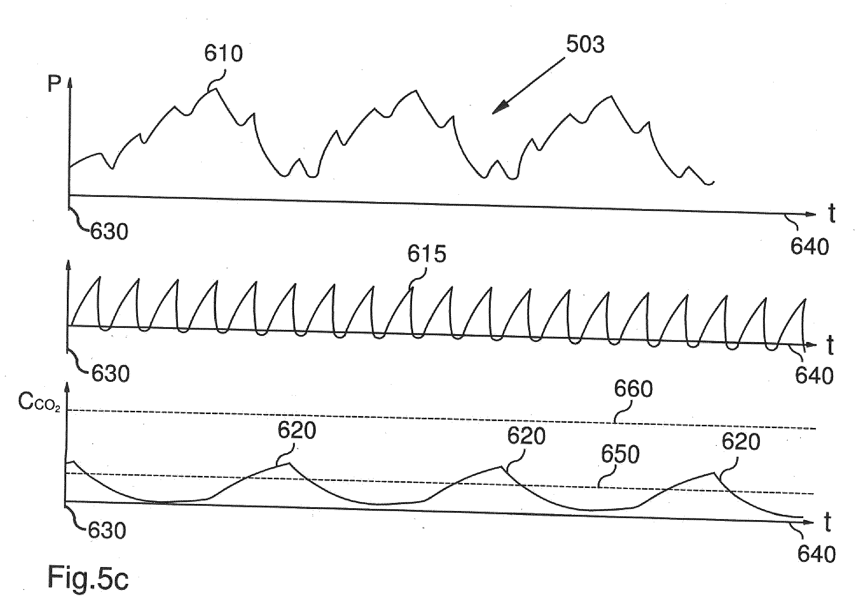

Das Alarmierungs- und Alarmanpassungseinheit 46 ist in einer technischen Umsetzung bevorzugt als Bestandteil der Steuer- und Regeleinheit 7 oder der Anzeige- und Signalgabeeinheit 4, bzw. als Teil des Betriebssystems des Beatmungsgerätes 1 (Figur 1a, 1b) ausgeführt. Es ist eine symbolische Störgröße Z 34 gezeigt, welche den Einfluss der Herz- Druck- Massage (HDM) in Form von Druckänderungen dP 35 auf die Druckmessung 13, sowie damit auf mittelbare Weise auch auf die Durchflussmessung 11, darstellt. Der durch die Herz- Druck- Massage (HDM) verursachte Effekt der Druckschwankungen auf das Signal des Beatmungsdrucks P 610 (Figuren 5a bis 5e) wird insbesondere aus den Darstellungen nach den Figuren 5c deutlich. Im Verfahren zum Betrieb eines Beatmungsgerätes zur Unterstützung der Herz- Lungen- Wiederbelebung (HLW) werden die Anpassungs- und Verzögerungselemente 22, 33 verwendet, um den Start der Inspirationsphase und den Start der Exspirationsphase dahingehend zu verzögern, die Herz- Druck- Massage (HDM) zu unterstützen. Die Unterstützung erfolgt in der Exspirationsphase dadurch, dass die Verzögerung durch das Exspiratorische Verzögerungselement 33 eine Erhöhung des anfänglichen exspiratorischen Druckniveaus und damit eine Vergrößerung eines mit Luft ausgefüllten Volumens der Lunge des Patienten bewirkt, so dass die Lunge einen Teil des Brustraumes ausfüllt und damit eine Ausweichmöglichkeit des Herzen im Brustraum bei einer Kompression durch die Herz-Druck- Massage (HDM) vermindert wird. Die Unterstützung erfolgt in der Inspirationsphase dadurch, dass die Verzögerung durch das Inspiratorische Verzögerungselement 22 eine Erniedrigung eines anfänglichen inspiratorischen Druckniveaus in der Lunge des Patienten, bzw. die Erzeugung eines geringen Unterdruck in der Lunge in Relation zum Umgebungsdruck erreicht, indem eine Zuführung von frischer Beatmungsluft am Beginn der Einatemphase durch eine Verzögerung der Öffnung des Inspirationsventils 2 zeitlich verzögert wird.
Damit wird bei Durchführung des Verfahrens zum Betrieb eines Beatmungsgerätes einer Unterstützung einer Herz- Lungen- Wiederbelebung (HLW) der Druck im Brustraum reduziert, so dass die Rückströmung des Blutes aus dem Körper zum Herzen bei einer Dekompression des Brustraumes unterstützt wird. Im Verfahren zum Betrieb eines Beatmungsgerätes zur Unterstützung der Herz- Lungen- Wiederbelebung (HLW) wird die Alarmierungs- und Alarmanpassungseinheit 46 verwendet, sodass Alarme, die durch die Herz- Druck- Massage (HDM) durch die Störgröße Z 34, z.B. als Druckänderungen dP 35, die das von der Beatmung hervorgerufene Signal des Beatmungsdrucks überlagert, ausgelöst würden, so behandelt werden, dass die Alarmgabe an den Anwender teilweise, zeitweiseweise oder vollständig für den Dauer des Verfahrens zum Betrieb eines Beatmungsgerätes mit einer Unterstützung einer Herz- Lungen- Wiederbelebung (HLW) unterdrückt werden. Auch kann die Störgröße in der Amplitude oder zeitlich gefiltert werden.

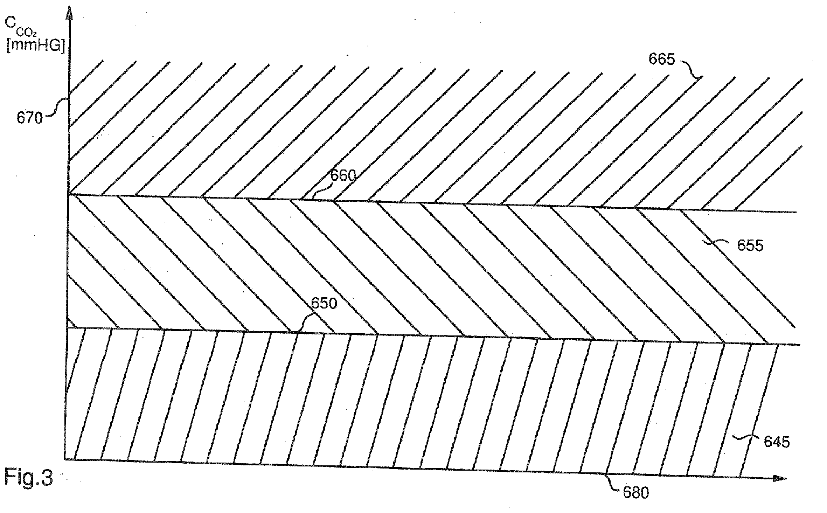

In der Figur 3 ist dargestellt, wie ein vorbestimmter erster 650 und ein vorbestimmter zweiter Schwellwert 660 der CO₂- Konzentration das Diagramm einer CO₂-Konzentration 506 in 3 Konzentrationsbereiche 645, 655, 665 einteilen. Auf einer Ordinate 670 ist die CO₂- Konzentration in der Einheit [mmHG] aufgetragen, auf einer Abszisse 680 ist der zeitliche Verlauf dimensionslos aufgetragen.

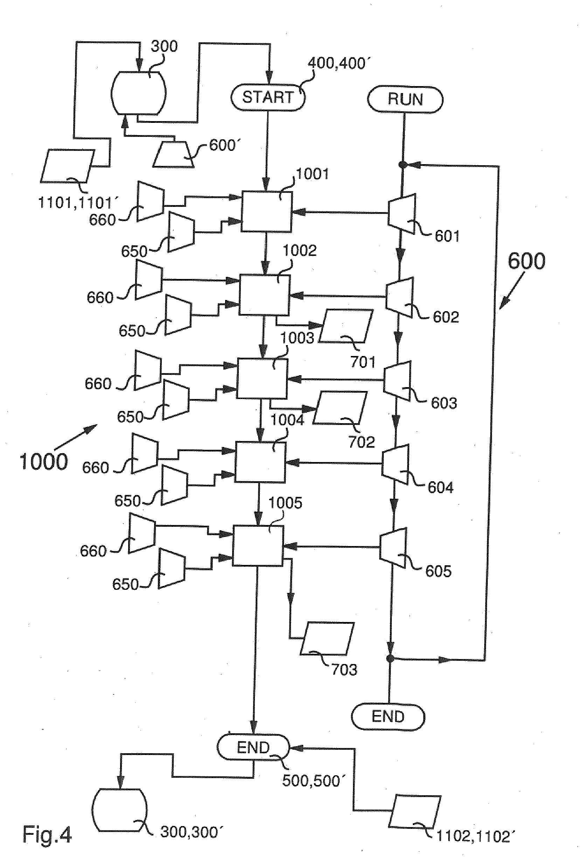

Es ergibt sich ein erster Konzentrationsbereich 645, in welchem die CO₂-Konzentration unterhalb des ersten vorbestimmten Schwellwertes 650 liegt. Die Unterschreitung dieses ersten vorbestimmten Schwellwertes 650 ist ein Indiz dafür, dass die Herz- Druck- Massage (HDM) nicht in ausreichender Weise durchgeführt wird, um die Herz- Kreislauffunktion des Patienten aufrechtzuerhalten. Es ergibt sich ein zweiter Konzentrationsbereich 655, in welchem die CO₂- Konzentration oberhalb des ersten vorbestimmten Schwellwertes 650 und unterhalb des zweiten vorbestimmten Schwellwertes 660 liegt. Die Unterschreitung des zweiten vorbestimmten Schwellwertes 660 ist ein Indiz dafür, dass der Patient nicht eigenständig in der Lage ist, seine Herz- Kreislauffunktion aufrechtzuerhalten und daher eine Herz- DruckMassage (HDM) durch den Anwender angewendet werden sollte. Es ergibt sich ein dritter Konzentrationsbereich 665, in welchem die CO₂- Konzentration oberhalb des zweiten vorbestimmten Schwellwertes 660 liegt. Die Überschreitung des zweiten vorbestimmten Schwellwertes ist ein Indiz dafür, dass der Patient eigenständig in der Lage, seine Herz- Kreislauffunktion aufrechtzuerhalten und daher keine Herz- DruckMassage (HDM) erforderlich ist, sowie, dass eine aktuell applizierte Herz- DruckMassage (HDM) durch den Anwender beendet werden sollte. Für einen praktischen Einsatz des Beatmungsgerätes 1, 1' (Figur 1a, Figur 1b) mit Unterstützung der Herz-Lungen- Wiederbelebung (HLW) in der Notfallmedizin sind ein erster vorbestimmter Schwellwert 650 von 10 mmHg mit einer Schwankungsbreite von +/- 2 mmHg und ein zweiter vorbestimmter Schwellwert 660 von 40 mmHg mit einer Schwankungsbreite von +/- 2 mmHg geeignete Werte. Bei der Unterschreitung und der Überschreitung des ersten vorbestimmten 650 und des zweiten vorbestimmten Schwellwertes 660 können zur Auslösung der Meldungen (Figur 4) an den Anwender, zum Start des Betriebes der Beatmung mit Unterstützung der Herz- Lungen- Wiederbelebung (HLW) und zur Regelung (Figur 2) des Beatmungsdrucks (Figur 2) im Betrieb des Beatmungsgerätes mit Unterstützung der Herz- Lungen- Wiederbelebung (HLW) weitere und zusätzliche Kriterien herangezogen und miteinander verknüpft werden.

Solche weiteren und zusätzlichen Kriterien sind beispielsweise Messwerte und / oder Einstellparameter des Beatmungsgerätes oder Messwerte und /oder Einstellparameter eines Physiologischen Monitors. Als Einstellparameter eines Beatmungsgerätes seien hier beispielhaft eine Sauerstoffkonzentration genannt.

Als Messwerte eines Physiologischen Monitors seien hier neben der CO₂-Konzentration, ein Sauerstoffpartialdruck SPO₂ im Blut oder ein auf nicht invasive Weise erfasster Blutdruck (NBP) oder eine Herzfrequenz (HR) des Patienten beispielhaft genannt.

In der Figur 4 ist ein schematischer Ablauf 1000 mit Beginn und Ende des Verfahrens des Betriebs eines Beatmungsgerätes zur Unterstützung der Herz- Lungen-Wiederbelebung (HLW) gezeigt. Der Ablauf 1000 ist in einen zeitlichen Verlauf einer Beatmung eines Patienten 47 (Figuren 1a, 1b, 2) mit einer ursprünglich gewählten beliebigen Beatmungsform 300 eingegliedert, in welchem an dem Patienten 47 (Figuren 1, 2) keine Herz- Druck- Massage (HDM) durchgeführt wird.

Durch eine erste Anwender- Interaktion 1101 wird aus der beliebigen Beatmungsform 300 ein Start 400 des Verfahrens zum Betrieb des Beatmungsgerätes 1, 1' (Figur 1a, 1b) mit Unterstützung der Herz- Lungen- Wiederbelebung (HLW) ausgelöst. Die beliebige Beatmungsform 300 kann eine Druck- oder eine Volumen- kontrollierte Beatmungsform sein. Ein Physiologischer Sensor oder ein externes Überwachungsgerät 31, 31' (Figur 1a, 1b) liefert in der beliebigen Beatmungsform 300 aktuelle Messsignale 600' einer aktuellen Kohlendioxidkonzentration, wie auch im Verfahren zum Betrieb des Beatmungsgerätes 1, 1' (Figur 1a, 1b) mit Unterstützung der Herz- Lungen- Wiederbelebung (HLW) in einer kontinuierlich sich wiederholenden Folge 600 in vorbestimmten zeitlichen Abständen Messsignale 601, 602, 603, 604, 605 einer aktuellen Kohlendioxidkonzentration als Eingangsgröße für den Ablauf 1000 des Verfahrens zum Betrieb des Beatmungsgerätes 1 (Figur 1a, 1b) mit Unterstützung der Herz- Lungen- Wiederbelebung (HLW) geliefert werden. Der Start 400 des Verfahrens zum Betrieb des Beatmungsgerätes 1, 1' (Figur 1a, 1b) mit Unterstützung der Herz-Lungen- Wiederbelebung (HLW) kann durch die erste Anwender- Interaktion 1101 oder aber auch automatisch durch das Beatmungsgerät 1, 1' (Figur 1a, 1b) z.B. auf Basis der Messsignale 600' der aktuellen Kohlendioxid- Konzentration, ohne eine Anwender-Interaktion erfolgen. Alternativ kann ein Start 400' auch halb- automatisch durch das Beatmungsgerät 1, 1' (Figur 1a, 1b) z.B. auf Basis der Messsignale 600' der aktuellen Kohlendioxid- Konzentration, erfolgen, wobei der Start 400' vom Beatmungsgerät 1 (Figur 1a, 1b) über die kombinierte Anzeige-, Signalgabe- und Eingabeeinheit 54 (Figur 1a, 1b) vorgeschlagen wird und durch eine erste Anwender- Interaktion 1101' der Start 400' dann final quittiert wird und vom Beatmungsgerät 1, 1' (Figur 1a, 1b) dann in den Betrieb des Beatmungsgerätes 1, 1' (Figur 1a, 1b) mit Unterstützung der Herz-Lungen- Wiederbelebung (HLW) 1000 gewechselt wird. Im Ablauf 1000 werden die in der Abfolge 600 kontinuierlich fortlaufend erfassten Messsignale 601, 602, 603, 604, 605 der aktuellen Kohlendioxidkonzentration ausgewertet. Bei dieser Auswertung wird im Ablauf 1000 durch einen Vergleich überprüft, in welcher Relation die Kohlendioxidkonzentration zum ersten vorbestimmten Schwellwert 650 und in welcher Relation die Kohlendioxidkonzentration zum zweiten vorbestimmten Schwellwert 660 liegt. Der Ablauf 1000 ist in dieser Figur 4 beispielhaft und exemplarisch in Phasen 1001, 1002, 1003, 1004, 1005 aufgeteilt, in denen die aktuelle KohlendioxidKonzentration mit dem ersten vorbestimmten Schwellwert 650 und dem zweiten vorbestimmten Schwellwert 660 verglichen wird. Die zeitliche Lage der Phasen 1001, 1002, 1003, 1004, 1005 im Ablauf kann dabei, wie in dieser Figur 4 abgebildet, aufeinander folgend sein, es ist aber im Sinne der vorliegenden Erfindung mit umfasst, dass die Phasen 1001, 1002, 1003, 1004, 1005 ohne eine zeitliche Koordination oder eine Koordination untereinander zu einem beliebigen Zeitpunkt der Beatmung oder im Ablauf der Beatmung bei der Behandlung oder Notfall- Behandlung eines Patienten stattfinden können. Die zeitliche Dauer der einzelnen Phasen 1001, 1002, 1003, 1004, 1005 richtet sich dabei nach der Behandlungssituation, der Konstitution des Patienten und der Einschätzung des Anwenders diesbezüglich. Weiterhin ist die zeitliche Dauer der einzelnen Phasen 1001, 1002, 1003, 1004, 1005 mittelbar abhängig von der Wahl des ersten vorbestimmten Schwellwert 650 und des zweiten vorbestimmten Schwellwert 660, sowie der Relation der Schwellwerte 650, 660 zueinander. In der ersten Phase 1001 des Ablaufs 1000 ergibt der Vergleich, dass die aktuelle Kohlendioxid- Konzentration 601 oberhalb des ersten vorbestimmten Schwellwertes 650 und unterhalb des zweiten vorbestimmten Schwellwertes 660 und damit in einem zweiten Konzentrationsbereich der CO₂- Konzentration 655 (Figur 3) liegt. Dies wird als ein Indiz dafür ausgewertet, dass die Herz- Kreislauffunktion zur Versorgung der Organe mit Sauerstoff mittels der Herz- Druck- Massage (HDM) erfolgreich durchgeführt wird. In der zweiten Phase 1002 des Ablaufs 1000 ergibt der Vergleich, dass die aktuelle Kohlendioxid- Konzentration 602 unterhalb des ersten vorbestimmten Schwellwertes 650 und unterhalb des zweiten vorbestimmten Schwellwertes 660 und damit in einem ersten Konzentrationsbereich der CO₂- Konzentration 645 (Figur 3) liegt. Dies wird als ein Indiz dafür ausgewertet, dass die Herz- Druck- Massage (HDM) nicht in ausreichender Weise durchgeführt wird, die Herz- Kreislauffunktion des Patienten zu ersetzen und die lebenswichtigen Organe, insbesondere das Gehirn, mit einer ausreichenden Menge an Sauerstoff zu versorgen. Es wird eine erste Meldung 701 an den Anwender gegeben, dass die Herz- Druck- Massage (HDM) nicht ordnungsgemäß durchgeführt wird. In der dritten Phase 1003 des Ablaufs 1000 ergibt der Vergleich, dass die aktuelle Kohlendioxid- Konzentration 603 wieder oberhalb des ersten vorbestimmten Schwellwertes 650 und unterhalb des zweiten vorbestimmten Schwellwertes 660 und damit in einem zweiten Konzentrationsbereich der CO₂-Konzentration 655 (Figur 3) liegt. Dies wird als ein Indiz dafür ausgewertet, dass die Herz- Kreislauffunktion zur Versorgung der Organe mit Sauerstoff mittels der Herz-Druck- Massage (HDM) wieder erfolgreich durchgeführt wird. Es wird eine zweite Meldung 702 an den Anwender gegeben, dass die Herz- Druck- Massage (HDM) wieder ordnungsgemäß durchgeführt wird. In der vierten Phase 1004 des Ablaufs 1000 ergibt der Vergleich, dass die aktuelle Kohlendioxid- Konzentration 604 weiterhin oberhalb des ersten vorbestimmten Schwellwertes 650 und unterhalb des zweiten vorbestimmten Schwellwertes 660 liegt. In der fünften Phase 1005 des Ablaufs 1000 ergibt der Vergleich, dass die aktuelle Kohlendioxid- Konzentration 605 oberhalb des ersten vorbestimmten Schwellwertes 650 und oberhalb des zweiten vorbestimmten Schwellwertes 660 und damit in einem dritten Konzentrationsbereich der CO₂-Konzentration 665 (Figur 3) liegt. Dies wird als ein Indiz dafür ausgewertet, dass die Herz- Kreislauffunktion mit Versorgung der Organe mit Sauerstoff durch den Patienten wieder selbständig aufrechterhalten wird. Es wird eine dritte Meldung 703 an den Anwender gegeben, dass die Herz- Kreislauffunktion des Patienten wieder selbständig aufrechterhalten werden kann. Die fünfte Phase 1005 des Ablaufs geht damit, beispielhaft in dieser Figur 4, über in die Beendigung 500 des Verfahrens zum Betrieb des Beatmungsgerätes 1, 1' (Figur 1a, 1b) mit Unterstützung der Herz- Lungen-Wiederbelebung (HLW). In dieser Figur 4 wird durch eine zweite Anwender- Interaktion 1102 eine Beendigung 500 des Verfahrens zum Betrieb des Beatmungsgerätes 1, 1' (Figur 1a, 1b) mit Unterstützung der Herz- Lungen- Wiederbelebung (HLW) ausgelöst. Die Beatmung des eines Patienten 47 (Figuren 1, 2) wird dann mit einer ursprünglich gewählten beliebigen Beatmungsform 300 oder mit einer anderen beliebigen Beatmungsform 300' fortgesetzt. Die beliebige Beatmungsform 300, 300' kann eine Druck- oder eine Volumen- kontrollierte Beatmungsform sein. Die Beendigung 500 kann aber auch automatisch durch das Beatmungsgerät 1 (Figur 1a, 1b) ohne eine Anwender- Interaktion erfolgen, oder eine Beendigung 500' kann auch halbautomatisch durch das Beatmungsgerät 1, 1' (Figur 1a, 1b) erfolgen, wobei die Beendigung 500 vom Beatmungsgerät 1, 1' (Figur 1a, 1b) über die kombinierte Anzeige-, Signalgabe- und Eingabeeinheit 54 (Figur 1a, 1b) vorgeschlagen wird und durch eine Anwender- Interaktion 1102' die Beendigung 500' final quittiert wird und dann vom Beatmungsgerät 1, 1' (Figur 1a, 1b) dann der Wechsel in die beliebige Beatmungsform 300, 300' vorgenommen wird.

In den Figuren 5a bis 5e sind beispielhaft Darstellungen 501, 502, 503, 504, 505 der zeitlichen Verläufe einer maschinellen Beatmung eines Patienten mit gleichzeitig durchgeführter Herz- Druck- Druckmassage (HDM) gezeigt.

Gezeigt sind dabei in einem Koordinatensystem von einer Abszisse 640 und von drei Ordinaten 630 zeitlich synchron und horizontal übereinander die zeitlichen Verläufe von einem Beatmungsdruck P 610 des Patienten, von einer Abbildung zur Veranschaulichung, ob eine Herz- Druck- Druckmassage (HDM) appliziert wird, in der vorliegenden Erfindung als eine sogenannte HDM- Aktivität 615 bezeichnet, sowie von einer CO₂- Konzentration cCO₂620.

Der Beatmungsdruck P 610 des Patienten, in der medizinischen Praxis zumeist gemessen und skaliert in den Dimensionen [mmH₂O], bzw. [mBar] oder [hPa], die dimensionslose HDM- Aktivität 615, und die CO₂- Konzentration cCO₂620, in der medizinischen Praxis zumeist gemessen und skaliert in der Dimension [mmHG] sind für verschiedene Phasen einer typischen Notfall- Beatmungssituation gezeigt.

Die Kurvenverläufe 610, 620 und deren zeitliche Zuordnung zueinander entsprechen in den Figuren 5a bis 5e im Prinzip einer Umsetzung mit einem "CO₂- Sidestream Sensor" 31 (Figur 1a). Die zeitliche Zuordnung ist in den Figuren 5a bis 5e nur beispielhaft gezeigt, da in der Praxis eine Vielzahl von Randbedingungen, wie beispielsweise Länge der Zuleitungen 48, 48' (Figur 1a, 1b), Länge und Durchmesser der Absaugeleitung 32 (Figur 1a), das Zeitverhalten der beteiligten Komponenten und der Signalverarbeitung eine wesentliche Rolle spielen. Bei einer Verwendung eines "CO₂- Mainstream Sensor" 31' (Figur 1b) würden sich eine prinzipiell andere und in der zeitlichen Synchronität der Kurvenverläufe 610, 620 verbesserte zeitliche Zuordnung ergeben, da dabei andere Randbedingungen zum Tragen kommen.

Die Figuren 5a bis 5e werden in einer gemeinsamen Figurenbeschreibung näher erläutert. Dabei wird ein typischer Hergang einer Notfallsituation eines beatmeten Patienten mit der Notwendigkeit und der Anwendung einer zwischenzeitlichen Herz-Druck- Massage (HDM) oder Herz-Lungen-Wiederbelebung in den Signalverläufen 501, 502, 503, 504, 505 des Beatmungsdrucks 610 und der KohlendioxidKonzentration 620 und der Einfluss der Herz- Druck- Massage (HDM) 615 auf die Signalverläufe des Beatmungsdrucks 610 und der Kohlendioxid- Konzentration 620 vereinfacht und schematisch dargestellt.

In der Darstellung 501 nach Figur 5a wird eine maschinelle Beatmung angenommen, der Patient ist intubiert oder über eine Maske mit dem Beatmungsgerät 1, 1' (Figur 1a, 1b) verbunden und wird beatmet, ein Physiologisches Monitoring zur Erfassung der Vitalparameter des Patienten ist angeschlossen, als mindestens ein Vitalparameter wird die CO₂- Konzentration 620 messtechnisch erfasst. Dem Beatmungsgerät 1, 1' (Figur 1a, 1b) stehen Messwerte des Physiologischen Monitoring, u.a. auch der aktuelle Wert der CO₂- Konzentration 620 über eine Daten- Schnittstelle 30 (Figur 1a, 1b) zur Verfügung. Der Wert der CO₂- Konzentration 620 liegt im Mittel oberhalb eines ersten vorbestimmten Schwellwertes 650 im dritten Konzentrationsbereich der CO₂-Konzentration 665 (Figur 3), was aussagt, dass die Herz- Kreislauffunktion des Patienten eigenständig und stabil ist. Es ist keine Anwendung einer Herz- DruckMassage (HDM) 615 erforderlich und das Beatmungsgerät 1, 1' arbeitet in einer ersten Betriebsart.

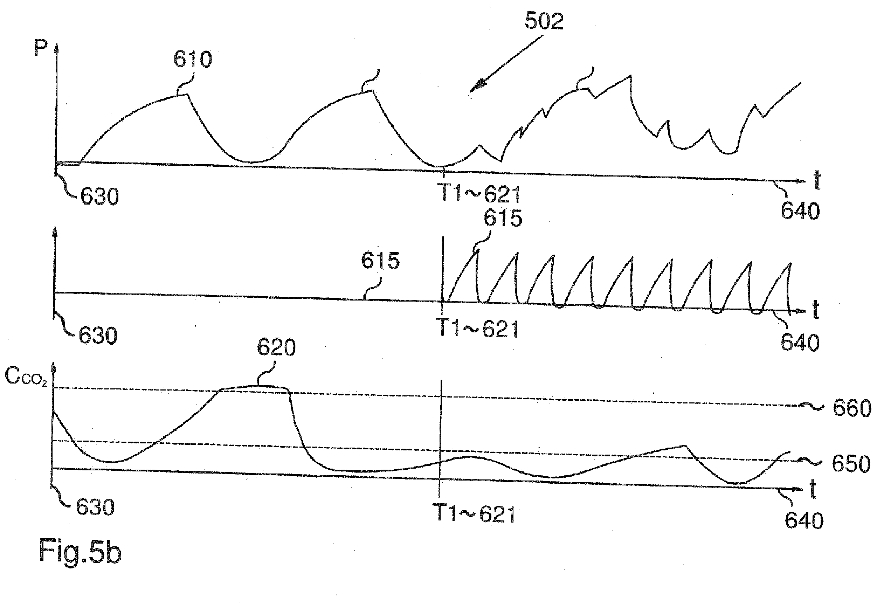

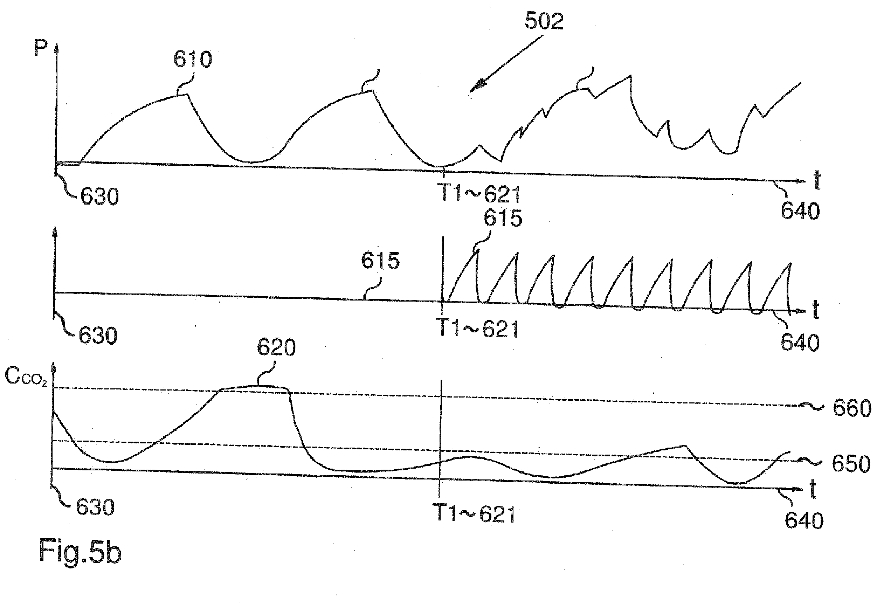

Die Figur 5b mit dem zeitlichen Verlauf 502 schließt sich in diesen beispielhaften Darstellungen der zeitlichen Verläufe 501, 502, 503, 504, 505 einer maschinellen Beatmung eines Patienten mit gleichzeitig durchgeführter Herz- Druck- Druckmassage (HDM) zeitlich an den Verlauf 501 der maschinellen Beatmung eines Patienten ohne gleichzeitig durchgeführte Herz- Druck- Druckmassage (HDM) nach der Figur 5a an.

In der Darstellung 502 nach Figur 5b sinkt zu einem Zeitpunkt T1 621 die CO₂-Konzentration 620 des Patienten im Verlauf der Beatmung unterhalb des ersten vorbestimmten Schwellwertes 650 in den ersten Konzentrationsbereich der CO₂-Konzentration 645 (Figur 3). Durch die Unterschreitung des ersten vorbestimmten Schwellwertes 650 ausgelöst, wird vom Beatmungsgerät 1, 1' (Figur 1a, 1b) eine Alarmierung (Figur 4) an den Anwender ausgegeben, dass die Herz- Kreislauffunktion des Patienten aktuell nicht gegeben ist. Zusätzlich wird im weiteren zeitlichen Verlauf an den Anwender eine Meldung (Figur 4) ausgegeben, dass das Beatmungsgerät einen Wechsel in den Betrieb eines Beatmungsgerätes mit einer Unterstützung einer Herz- Lungen- Wiederbelebung (HLW), also die zweite Betriebsart, vorbereitet hat und automatisch den Wechsel in den Betrieb des Beatmungsgerätes mit Unterstützung der Herz- Lungen- Wiederbelebung (HLW) vornehmen wird. Ein automatischer Wechsel in den Betrieb des Beatmungsgerätes mit Unterstützung der Herz- Lungen-Wiederbelebung (HLW), d.h. die zweite Betriebsart, kann dabei beispielsweise dadurch ausgelöst werden, wenn die Unterschreitung des ersten vorbestimmten Schwellwertes 650 der Kohlendioxid- Konzentration länger als eine vorbestimmte erste Zeitdauer andauert. Das Beatmungsgerät (Figur 1a, 1b) wechselt daraufhin automatisch in den Betrieb des Beatmungsgerätes mit einer Unterstützung der Herz- Lungen-Wiederbelebung (HLW) (zweite Betriebsart) und gibt eine entsprechende Meldung (Figur 4) aus. Der Anwender beginnt mit der Herz- Druck- Massage (HDM) und im zeitlichen Verlauf des Beatmungsdrucks zeitlich nach dem ersten Zeitpunkt T1 621 ist der Einfluss der Herz- Druck- Massage (HDM) in Form einer Überlagerung von Druckspitzen mit der Frequenz der Herz- Druck- Massage (HDM) ersichtlich.

Die Figur 5c mit dem zeitlichen Verlauf 503 schließt sich in diesem beispielhaften Darstellungen der zeitlichen Verläufe 501, 502, 503, 504, 505 einer maschinellen Beatmung eines Patienten mit gleichzeitig durchgeführter Herz- Druck- Druckmassage (HDM) (zweite Betriebsart) zeitlich an den Verlauf 502 der maschinellen Beatmung eines Patienten mit gleichzeitig durchgeführter Herz- Druck- Druckmassage (HDM) nach der Figur 5b an.

In der Darstellung 503 nach Figur 5c dauert die in der Figur 5b begonnenen Herz-Druck- Massage (HDM) unter Beibehaltung der maschinellen Beatmung an. Die CO₂-Konzentration des Patienten steigt aber bedingt durch die Herz- Druck- Massage (HDM) im Verlauf der Herz- Druck- Massage (HDM) oberhalb des ersten vorbestimmten Schwellwertes 650 an. Die CO₂- Konzentration des Patienten liegt dabei jedoch weiterhin unterhalb des zweiten vorbestimmten Schwellwertes 660 im zweiten Konzentrationsbereich der CO₂- Konzentration 655 (Figur 3), was ein Indiz dafür ist, dass die Beatmung und die Herz- Druck- Massage (HDM) in einer Form durchgeführt werden, dass sowohl die Lungenventilation, durch die Beatmung, als auch die Herz- Kreislauffunktion zur Versorgung der Organe mit Sauerstoff mittels der Herz- Druck- Massage (HDM) aufrecht erhalten werden, aber der Patient die HerzKreislauffunktion noch nicht eigenständig übernehmen und aufrechterhalten kann. Im zeitlichen Verlauf des Beatmungsdrucks 610 ist der Einfluss der Herz- Druck- Massage (HDM) in Form einer Überlagerung von Druckspitzen mit der Frequenz der Herz-Druck- Massage (HDM) ersichtlich.

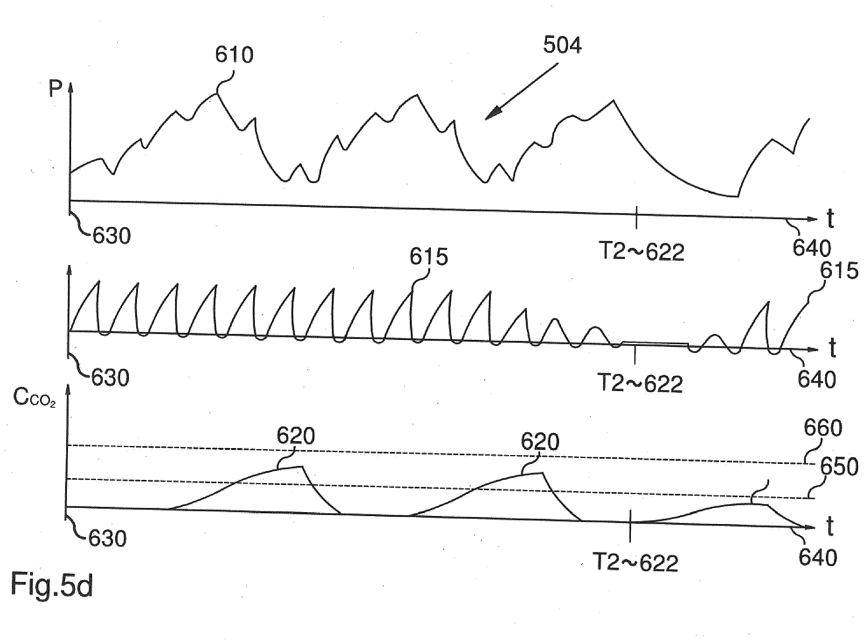

Die Figur 5d mit dem zeitlichen Verlauf 504 schließt sich in diesem beispielhaften Darstellungen der zeitlichen Verläufe 501, 502, 503, 504, 505 einer maschinellen Beatmung eines Patienten mit gleichzeitig durchgeführter Herz- Druck- Druckmassage (HDM) zeitlich an den Verlauf 503 der maschinellen Beatmung eines Patienten mit gleichzeitig durchgeführter Herz- Druck- Druckmassage (HDM) nach der Figur 5c an.

In der Darstellung 504 nach Figur 5d ist im zeitlichen Verlauf eine Beatmung mit gleichzeitiger Herz- Druck- Massage (HDM) gezeigt, bei dem die CO₂- Konzentration 620 des Patienten zeitweise unterhalb des ersten vorbestimmten Schwellwertes 650 im ersten Konzentrationsbereich der CO₂- Konzentration 645 (Figur 3) liegt. Bei Unterschreitung des ersten vorbestimmten Schwellwertes 650 zu einem zweiten Zeitpunkt T2 622 wird durch das Beatmungsgerät 1, 1' (Figur 1a, 1b) im Verfahren zum Betrieb eines Beatmungsgerätes 1, 1' (Figur 1a, 1b) eine Meldung (Figur 4) an den Anwender ausgegeben, dass die Herz- Druck- Massage (HDM) nicht in ausreichender Weise durchgeführt wird, die Herz- Kreislauffunktion des Patienten zu ersetzen und die lebenswichtigen Organe, insbesondere das Gehirn, mit einer ausreichenden Menge an Sauerstoff zu versorgen. Das ist beispielsweise gegeben, wenn die Druckmassage (HDM) nicht mit genügend Druck ausgeübt wird oder die zeitlichen Abstände zwischen den einzelnen Verabreichungen der Druckmassage (HDM) auf den Brustkorb des Patienten zu groß sind oder die Herz- Druck- Massage (HDM) insgesamt unregelmäßig durchgeführt wird. Auch am zeitlichen Verlauf des Beatmungsdrucks 610 ist zum zweiten Zeitpunkt T2 622 ersichtlich, dass die Herz- Druck- Massage (HDM) 615 nicht ordnungsgemäß durchgeführt wird, da die Überlagerung von Druckspitzen mit der Frequenz der Herz- Druck- Massage (HDM) nicht mehr ersichtlich ist. Im weiteren Verlauf dieser Darstellung zeitlich im Anschluss an den zweiten Zeitpunkt T2 622 wird Herz- Druck- Massage (HDM) 615 insgesamt wieder ordnungsgemäß durchgeführt.

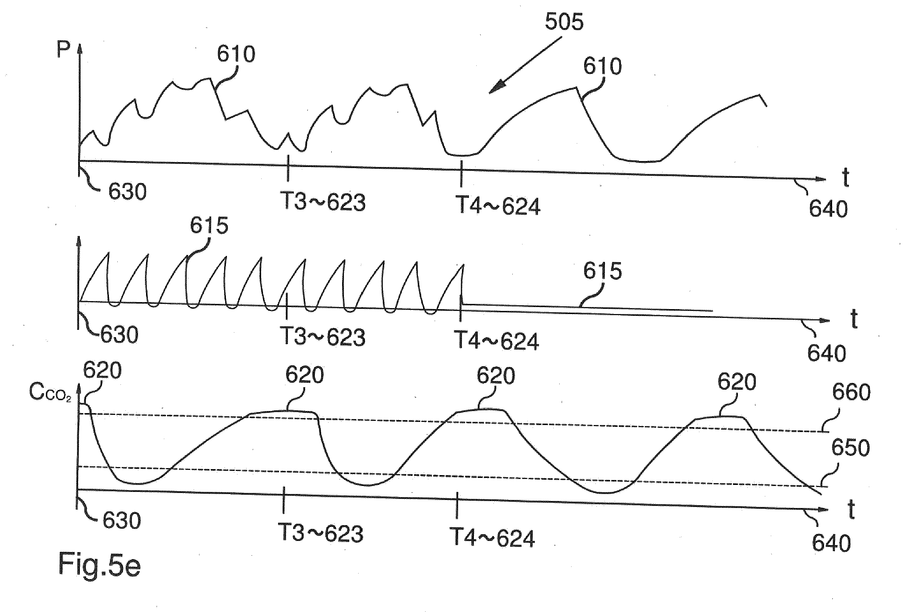

Die Figur 5e mit dem zeitlichen Verlauf 505 schließt sich in diesem beispielhaften Darstellungen der zeitlichen Verläufe 501, 502, 503, 504, 505 einer maschinellen Beatmung eines Patienten mit gleichzeitig durchgeführter Herz- Druck- Druckmassage (HDM) zeitlich an den Verlauf 504 der maschinellen Beatmung eines Patienten mit gleichzeitig durchgeführter Herz- Druck- Druckmassage (HDM) nach der Figur 5d an.

In der Darstellung 505 nach Figur 5e steigt im zeitlichen Verlauf zu einem dritten Zeitpunkt T3 623 die CO₂- Konzentration des Patienten im zeitlichen Verlauf oberhalb des zweiten vorbestimmten Schwellwertes 660 in den dritten Konzentrationsbereich der CO₂- Konzentration 665 an, was ein Indiz dafür ist, dass die Herz- Kreislauffunktion wieder vom Patienten selbst stabil aufrecht erhalten werden kann. Im weiteren Verlauf zu einem vierten Zeitpunkt T4 624, zeitlich im Anschluss an den dritten Zeitpunkt T3 623 wird die Herz- Druck- Druckmassage (HDM) beendet. Die Tatsache, dass die CO₂-Konzentration des Patienten im weiteren zeitlichen Verlauf im Anschluss an den vierten Zeitpunkt T4 624 oberhalb des zweiten vorbestimmten Schwellwertes 660 verbleibt, kann als weiteres Indiz gewertet werden, dass der Patient seine Herz- Kreislauffunktion nun wieder selbständig stabil aufrechterhalten kann.

Im Ablauf 1000 (Figur 4) der Beatmung unter zeitweiliger Anwendung der Herz- Druck-Druckmassage (HDM) nach den Figuren 5a bis 5e werden im Betrieb des Beatmungsgerätes mit Unterstützung der Herz- Lungen- Wiederbelebung (HLW) einige Alarmierungsgrenzen des Beatmungsgerätes 1, 1' (Figur 1a, 1b) durch entsprechende Mittel zur Anpassung 46 (Figur 2) in einer Weise verstellt oder ausgeblendet, bzw. die akustische wird vorzugsweise Alarmierung "stumm- geschaltet", so dass der Anwender bei der die Beatmung, bei der Anwendung der die Herz- Druck Massage (HDM) und bei der Behandlung des Patienten nicht in störender Weise beeinträchtigt wird.

Weiterhin ist die Überwachung der Überschreitung und Unterschreitung des ersten vorbestimmten Schwellwertes 650 und Überwachung der Überschreitung und Unterschreitung des zweiten vorbestimmten Schwellwertes 660 vorzugsweise mit einer Filterung versehen, so dass die bei Überschreitung und Unterschreitung ausgelösten Wechsel in oder aus dem Betrieb des Beatmungsgerätes mit einer Unterstützung der Herz- Lungen- Wiederbelebung (HLW) und die Meldungen (Figur 4) an den Anwender nicht in zu häufiger Folge stattfinden können. Diese Filterung kann beispielsweise als Amplitudenfilterung, Mittelwertfilterung oder Medianfilterung direkt auf dem Wert der CO₂- Konzentration oder davon abgeleiteten Werten vorgenommen werden, ebenso ist eine zeitliche Filterung mit einem Überwachungszeitfenster möglich. Das Überwachungszeitfenster kann beispielsweise derart gestaltet sein, dass Wertänderungen der CO₂- Konzentration für eine gewisse Zeit andauern müssen, bevor Wechsel in oder aus dem Betrieb des Beatmungsgerätes mit einer Unterstützung der Herz- Lungen- Wiederbelebung (HLW) durchgeführt werden oder Meldungen (Figur 4) an den Anwender gegeben werden. Neben der Anpassung der Ausgabe von Meldungen (Fig. 4) im Betrieb des Beatmungsgerätes mit Unterstützung der Herz- Lungen- Wiederbelebung (HLW) ist es weiterhin vorteilhaft, dass während einer Druck- kontrollierten Beatmungsform, wie beispielsweise CPAP, BiPAP, PC-PPS, PC-PS, die Regelung nicht auf die Änderungen im gemessenen Beatmungsdruck (Figur 2) reagiert, welche durch die Herz- Druck- Massage (HDM) hervorgerufen werden. Dazu wird im Betrieb des Beatmungsgerätes 1, 1' (Figur 1a, 1b) mit Unterstützung der Herz- Lungen- Wiederbelebung (HLW) durch geeignete Anpassungs- und Verzögerungselemente 33, 22 (Figur 2) in vorteilhafter Weise eine Filterung und / oder Verzögerung des gemessenen Beatmungsdrucks vorgenommen. Diese Filterung kann beispielsweise als Amplitudenfilterung, Mittelwertfilterung oder Medianfilterung direkt auf dem gemessen Wert des Beatmungsdrucks (Figur 2) oder davon abgeleiteten Werten, sowie auch im weiteren Verlauf der Signalverarbeitung, beispielsweise vor oder in einer Rückführung in den Regelkreis vorgenommen werden. Ebenso ist eine zeitliche Filterung mit einem Überwachungszeitfenster möglich. Das Überwachungszeitfenster kann beispielsweise derart gestaltet sein, dass Wertänderungen dP 35 (Figur 2) des Beatmungsdrucks für eine gewisse Zeit andauern müssen, bevor eine Nachregelung (Figur 2) des Beatmungsdrucks durch den Regelkreis erfolgt. Dadurch wird ein Anschwingen, ein Einschwingen oder ein Überschwingen oder gar ein Aufschwingen des Beatmungsdrucks als Reaktion auf die Herz- Druck- Massage (HDM) verhindert.

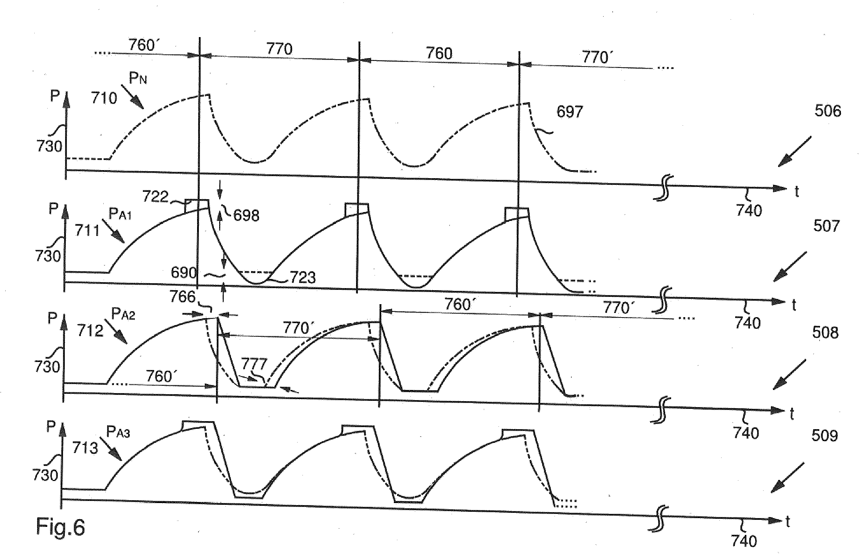

Die Figur 6 zeigt in Darstellungen 506, 507, 508, 509 jeweils in einem Koordinatensystem von einer Abszisse 740 und von einer Ordinate 730 Zeitverläufe einer Beatmung eines Patienten bei Betrieb eines Beatmungsgerätes mit und ohne eine Unterstützung einer Herz- Lungen- Wiederbelebung (HLW) (erste und zweite Betriebsart). Aus der Darstellung ist eine Variation des Beatmungsdrucks bei Betrieb des Beatmungsgerätes mit Unterstützung einer Herz- Lungen- Wiederbelebung (HLW) (zweite Betriebsart) gegenüber dem Betrieb des Beatmungsgerätes ohne Unterstützung einer Herz- Lungen- Wiederbelebung (HLW) (erste Betriebsart) ersichtlich. In der Darstellung 506 ist als eine Kurve in gestrichelter Linie ein normaler zeitlicher Verlauf von einem Beatmungsdruck P_{N} 710 eines maschinell beatmeten Patienten im Wechsel von Einatmung zu Ausatmung, wie er sich im normalen Betrieb eines Beatmungsgerätes ohne eine Unterstützung einer Herz- Lungen-Wiederbelebung (HLW) ergibt. Im normalen Betrieb des Beatmungsgerätes 1, 1' (Figur 1a, 1b) ohne Unterstützung der Herz- Lungen- Wiederbelebung (HLW) (erste Betriebsart) wird der Beatmungsdruck P_{N} 710 durch eine Steuerungs- und Regeleinheit 7 (Figur 1a, 1b) dadurch geregelt, dass ein durch einen Drucksensor 13 (Figur 1a, 1b) erfasster aktueller Wert 610 (Figur 4) des Beatmungsdrucks durch einen Vergleich mit einem ersten vorbestimmten Wert 697, 16 (Figur 1a, 1b) einen Stellwert zu einer Ansteuerung ergibt und die Steuerungs- und Regeleinheit 7 (Figur 1a, 1b) auf Basis des Vergleichs ein Exspirationsventil 3 (Figur 1a, 1b) und ein Inspirationsventil 2 (Figur 1a, 1b) so ansteuert, dass der aktuelle Wert 610 des Beatmungsdrucks dem ersten vorbestimmten Wert 697, 16 (Figur 1a, 1b) im Wesentlichen entspricht. Der erste vorbestimmte Wert ist dabei im Sinne der vorliegenden Erfindung nicht nur ein einzelner Wert, auf welchen die Steuerungs- und Regeleinheit 7 (Figur 1a, 1b) regelt, sondern eine zeitliche Abfolge von vorbestimmten Soll- Werten, z.B. in Form einer Beatmungskurve oder eines Verlaufs zur Steuerung der Beatmung. In der Figur 6 in der Darstellung 507 ist als eine Kurve in durchgezogener Linie ein erster alternativer zeitlicher Verlauf 711 eines Beatmungsdrucks P_{A1} eines maschinell beatmeten Patienten im Wechsel von Einatmung zu Ausatmung gezeigt, wie er sich im Betrieb eines Beatmungsgerätes mit Unterstützung einer Herz- Lungen- Wiederbelebung (HLW) (zweite Betriebsart) ergibt. Der Verlauf des normalen Beatmungsdrucks 710 nach der Darstellung 506 ist als Kurve in gestrichelter Linie in dieser Darstellung 507 zur Hervorhebung der Unterschiede zwischen dem ersten alternativen zeitlichen Verlauf 711 und dem normalen zeitlichen Verlauf 710 mit dargestellt.

Im Betrieb des Beatmungsgerätes 1, 1' (Figur 1a, 1b) mit Unterstützung der Herz-Lungen- Wiederbelebung (HLW) wird der Beatmungsdruck P_{A} 711 in einer ersten und zweiten Phase (760, 770) der Beatmung durch die Steuerungs- und Regeleinheit 7 (Figur 1a, 1b), ebenso wie zuvor für den normalen Betrieb des Beatmungsgerätes 1, 1' (Figur 1a, 1b) beschrieben, auf den ersten vorbestimmten Wert 697, 16 (Figur 1a, 1b) ausgeregelt, mit der Besonderheit, dass von der Steuerungs- und Regeleinheit 7 (Figur 1a, 1b) das Exspirationsventil 3 (Figur 1a, 1b) und das Inspirationsventil 2 (Figur 1a, 1b) in besonderer Weise so angesteuert werden, dass sich am Anfang der ersten Phase 760 der Beatmung, der Exspirationsphase, ein um einen zweiten vorbestimmten Wert 698erhöhter Druck P_{H} 722 ergibt und, in dass sich am Anfang der zweiten Phase 770 der Beatmung, der Inspirationsphase, ein um einen dritten vorbestimmten Wert 699 erniedrigter Druck P_{L} 723 ergibt. Zusätzlich ist in der Figur 6 in einer Darstellung 508 als eine Kurve in durchgezogener ein zweiter alternativer zeitlicher Verlauf 712 eines Beatmungsdrucks P_{A2} eines maschinell beatmeten Patienten im Wechsel von Einatmung zu Ausatmung gezeigt, wie er sich im Betrieb eines Beatmungsgerätes mit Unterstützung einer Herz- Lungen- Wiederbelebung (HLW) (zweite Betriebsart) ergibt. Der Verlauf des normalen Beatmungsdrucks 710 nach der Darstellung 506 ist als Kurve in gestrichelter Linie in dieser Darstellung 508 zur Hervorhebung der Unterschiede zwischen dem zweiten alternativen zeitlichen Verlauf 712 und dem normalen zeitlichen Verlauf 710 mit dargestellt. Im Betrieb des Beatmungsgerätes 1, 1' (Figur 1a, 1b) mit Unterstützung der Herz- Lungen- Wiederbelebung (HLW) (zweite Betriebsart) wird der Beatmungsdruck P_{A} 712 in einer ersten und zweiten Phase (760, 770) der Beatmung durch die Steuerungs- und Regeleinheit 7 (Figur 1a, 1b), ebenso wie zuvor für den normalen Betrieb des Beatmungsgerätes 1, 1' (Figur 1a, 1b) beschrieben, auf den ersten vorbestimmten Wert 697, 16 (Figur 1a, 1b) ausgeregelt, mit der Besonderheit, dass von der Steuerungs- und Regeleinheit 7 (Figur 1a, 1b) das Exspirationsventil 3 (Figur 1a, 1b) und das Inspirationsventil 2 (Figur 1a, 1b) in besonderer Weise so angesteuert werden, dass sich am Anfang der ersten Phase 760 der Beatmung, der Exspirationsphase, eine um einen zweiten vorbestimmten Zeit-Wert 766 zeitlich verlängerte Phase erste 760' ergibt und, dass sich am Anfang der zweiten Phase 770 der Beatmung, der Inspirationsphase, eine um einen dritten vorbestimmten Zeit- Wert 777 zeitlich verschobene zweite Phase 770' ergibt. Weiterhin ist in der Figur 6 in einer Darstellung 509 als eine Kurve in durchgezogener ein dritter alternativer zeitlicher Verlauf 713 eines Beatmungsdrucks P_{A3} eines maschinell beatmeten Patienten gezeigt, wie er sich aus einer Kombination des ersten alternativen Verlaufs 711 aus der Darstellung 507 und des zweiten Alternativen Verlaufs 712 aus der Darstellung 508 im Betrieb eines Beatmungsgerätes mit Unterstützung einer Herz- Lungen- Wiederbelebung (HLW) ergibt. Der Verlauf des normalen Beatmungsdrucks 710 nach der Darstellung 506 ist als Kurve in gestrichelter Linie in dieser Darstellung 509 zur Hervorhebung der Unterschiede zwischen dem dritten alternativen zeitlichen Verlauf 713 und dem normalen zeitlichen Verlauf 710 mit dargestellt.

Die Figur 7a zeigt in einer schematischen Übersicht ein Medizinisches System 1590 mit einem Beatmungsgerät 1, 1' nach Figur 1a oder Figur 1b und mit einem Unterstützungsgerät 1500 zur automatischen Durchführung einer Herzdruckmassage (HDM). Das Unterstützungsgerät 1500 ist mittels eines Kompressionselementes 1550 mit einem Brustkorb 1470 eines Patienten 47 verbunden. Das Unterstützungsgerät 1500 ist über eine Sensor- und Daten- Schnittstelle 30 mit dem Beatmungsgerät 1, 1' verbunden. Das Unterstützungsgerät 1500 leitet über die Sensor- und DatenSchnittstelle 30 ein Steuersignal 1560 an das Beatmungsgerät 1,1' um das Beatmungsgerät 1, 1' in einen Pausenzustand zu versetzen oder einen Betrieb des Beatmungsgerätes 1, 1' mit einer Unterstützung einer Herz- Lungen- Wiederbelebung (HLW) zu starten oder zu beenden oder um eine Änderung einer Alarmgebung am Beatmungsgerät 1, 1' zu bewirken. Das Beatmungsgerät 1, 1' ist in der Lage, mittels eines weiteren Steuersignals 1550 das Unterstützungsgerät 1500 in einen Pausenzustand zu versetzen.

Die Figur 7b zeigt schematisch ein Medizinisches System 1690 mit einem Beatmungsgerät 1, 1' nach Figur 1 a oder Figur 1b und mit einem zu einer Reanimation geeigneten Spannungsgenerator / Defibrillator 1600. Der Spannungsgenerator 1600 ist mittels Elektroden 1650 mit einem Brustkorb 1470 eines Patienten 47 verbunden. Der Spannungsgenerator 1600 ist über eine Sensor- und Daten- Schnittstelle 30 mit dem Beatmungsgerät 1, 1' verbunden. Der Spannungsgenerator 1600 leitet über die Sensor- und Daten- Schnittstelle 30 ein Steuersignal 1660 an das Beatmungsgerät 1,1' um das Beatmungsgerät 1, 1' in einen Pausenzustand zu versetzen oder eine um eine Änderung einer Alarmgebung am Beatmungsgerät 1, 1' zu bewirken.

### Bezuasziffernliste

- 1: Beatmungsgerät
- 2: Inspiratorische Dosiereinheit, Inspirationsventil,
- 3: Exspiratorische Dosiereinheit, Exspirationsventil
- 4: Anzeige- und Signalgabeeinheit
- 5: Eingabeeinheit zur Parametereingabe
- 6: Stellgrößeneingang
- 7: Steuer- und Regeleinheit
- 8: Spannungsversorgungseinheit
- 9: Gasmisch- und Dosiereinheit
- 10: Darstellung von Komponenten des Beatmungsgerät nach Figur 1
- 11: Durchflussmessung / Durchflusssensor
- 12: Druck- und Durchflussregelung
- 13, 13': exspiratorische Druckmessung / Drucksensor
- 14: Sauerstoffdruckflasche
- 15: Druckreduziereinheit
- 16: Einstellgrößen
- 17: Verbindungsstück
- 21: Daten
- 22: Anpassungs- und Verzögerungselement Inspiratorisch
- 23: inspiratorische Druckmessung / Drucksensor
- 24: Patientennahe Durchflussmessung / Flowsensor
- 27: Gebläseeinheit, Beatmungsantrieb
- 29: Sauerstoff- Luft- Misch und Dosierventil
- 30: Daten- Schnittstelle
- 31, 31': Physiologischer Sensor, Kohlendioxid Sensor
- 32: Absaugeleitung
- 33: Anpassungs- und Verzögerungselement exspiratorisch
- 34: Störgröße Z (HDM /CPR)
- 35: Druckänderung dP
- 36: Datenleitung
- 37: CO₂- Analysegerät
- 38: Beatmungsverlauf
- 39: erster Atemzyklus
- 40: folgende Atemzyklen
- 44: akustisches Signalmittel (Hupe, Sirene, Lautsprecher)
- 45: optisches Signalmittel
- 46: Alarmierungs- und Alarmanpassungseinheit
- 47: Patient
- 48: Zuleitungen / Schlauchsystem
- 50: Toleranzbereich
- 54: kombinierte Anzeige-, Signalgabe- und Eingabeeinheit
- 55: Schaltelement, Taster
- 88: Batterien, Speicher für elektrische Energie
- 89: Lade- und Versorgungselement
- 90: Schnittstelle für elektrische Energie
- 91: Inspiratorischer Gasanschluss
- 92: Exspiratorischer Gasanschluss
- 93: Gasauslass
- 94: Ejektor
- 95: Luft- Gaszuführung
- 96: Sauerstoff- Gaszuführung
- 300, 300': Beliebige Beatmungsform
- 400: Start des Verfahrens zur Unterstützung der Herz- Lungen- Wiederbelebung (HLW)
- 500: Beendigung des Verfahrens zur Unterstützung der Herz- Lungen- Wiederbelebung (HLW)
- 501 - 506: Darstellungen der zeitlichen Verläufe einer maschinellen Beatmung eines Patienten
- 600: Abfolge von Messsignale
- 601- 605: Messsignale der Kohlendioxid- Konzentration
- 610: Verlauf des Beatmungsdrucks
- 615: Verlauf der HDM- Aktivität
- 620: Verlauf der der CO₂- Konzentration
- 621: erster Zeitpunkt T1
- 622: zweiter Zeitpunkt T2
- 623: dritter Zeitpunkt T3
- 624: vierter Zeitpunkt T4
- 630: Ordinate
- 640: Abszisse, zeitlich skaliert
- 645: erster Konzentrationsbereich der CO₂- Konzentration
- 650: erster vorbestimmter Schwellwert
- 655: zweiter Konzentrationsbereich der CO₂- Konzentration
- 660: zweiter vorbestimmter Schwellwert
- 665: dritter Konzentrationsbereich der CO₂- Konzentration
- 670: Ordinate
- 680: Abszisse, zeitlich unskaliert
- 697: erster vorbestimmter Wert (Druck)
- 698: zweiter vorbestimmter Wert (Druck)
- 699: dritter vorbestimmter Wert (Druck)
- 701: erste Meldung
- 702: zweite Meldung
- 703: dritte Meldung
- 710: Normaler zeitlicher Verlauf des Beatmungsdrucks P_{N}
- 711: Erster alternativer zeitlicher Verlauf des Beatmungsdrucks P_{A}
- 712: Zweiter alternativer zeitlicher Verlauf des Beatmungsdrucks P_{A}
- 713: Dritter alternativer zeitlicher Verlauf des Beatmungsdrucks P_{A}
- 722: erhöhter Beatmungsdruck P_{H}
- 723: erniedrigter Beatmungsdruck P_{L}
- 740: Abszisse
- 730: Ordinate
- 760, 760': erste Phase der Beatmung
- 766: zweiter vorbestimmter Zeit- Wert
- 770, 770': zweite Phase der Beatmung
- 777: dritte vorbestimmte Zeit-Wert
- 1000: Ablauf des Verfahrens zur Unterstützung der Herz- Lungen- Wiederbelebung (HLW)
- 1001- 1005: Phasen des Ablaufs 1000 des Verfahrens zur Unterstützung der Herz- Lungen- Wiederbelebung (HLW)
- 1101: erste Anwender- Interaktion
- 1102: zweite Anwender- Interaktion
- 1470: Brustkorb des Patienten 47
- 1500: Unterstützungsgerät zur automatischen Durchführung einer Herzdruckmassage (HDM)
- 1550, 1560, 1660: Steuersignale
- 1580: Kompressionselement
- 1600: Spannungsgenerator / Defibrillator
- 1650: Elektroden
- 1490, 1590, 1690: Varianten eines Medizinischen Systems

## Patentansprüche

1. Beatmungssystem mit einer Gasversorgungseinrichtung
mit einer Schlauchanordnung (48, 91, 92), wobei die Schlauchanordnung (48, 91, 92) einen Patientenanschluss (17) zur Verbindung mit einem Patienten aufweist, um Gas von der Gasversorgungseinrichtung (9) zum Patienten zu führen und um vom Patienten ausgeatmetes Gas abzuführen,
mit Mitteln zum Steuern des Gasstromes (2, 3) von der Gasversorgungseinrichtung (9) zum Patientenanschluss (17) und zum Steuern des Gasstroms vom Patientenanschluss (17) weg,
mit einer Sensoreinheit (13, 23, 31, 31'), die derart in der Schlauchanordnung (48, 91, 92) angeordnet und eingerichtet ist, Parameter des dem Patienten zugeführten und vom Patienten ausgeatmeten Gases zu erfassen,
mit einer Steuer- und Regeleinheit (7) zum Steuern der Gasversorgungseinrichtung (9) und der Mittel zum Steuern des Gasstroms (2, 3), die mit der Gasversorgungseinrichtung (9), den Mitteln zum Steuern des Gasstroms (2, 3) und der Sensoreinheit (13, 23, 31, 31'), verbunden ist,
wobei die Steuer- und Regeleinheit (7) derart ausgestaltet ist, dass in einer ersten Betriebsart während einer Exspirationsphase die Mittel zum Steuern des Gasstroms (2, 3) durch die Steuer- und Regeleinheit (7) derart angesteuert werden, dass der Druck am Patientenanschluss (17) des vom Patienten ausgeatmeten Gases einen ersten Verlauf hat, und
während einer Inspirationsphase die Mittel zum Steuern des Gasstroms (2, 3) durch die Steuer- und Regeleinheit (7) derart angesteuert werden, dass der Druck am Patientenanschluss (17) des dem Patienten zugeführten Gases einen zweiten Verlauf hat,
wobei die Exspirationsphase und die Inspirationsphase kontinuierlich alternierend aufeinander folgen,
wobei die Steuer- und Regeleinheit (7) ausgestaltet ist, eine zweite Betriebsart zu haben,
wobei im Beatmungssystem Mittel vorgesehen sind, die Steuer- und Regeleinheit (7) zwischen der ersten und der zweiten Betriebsart umzuschalten, und
wobei die Steuer- und Regeleinheit (7) derart ausgestaltet ist, dass in der zweiten Betriebsart während der Exspirationsphase die Mittel zum Steuern des Gasstroms (2, 3) durch die Steuer- und Regeleinheit (7) derart angesteuert werden, dass die Ansteuerung in der Exspirationsphase zwei aufeinanderfolgende Teilphasen aufweist, wobei in der ersten Teilphase der Exspirationsphase der Druck am Patientenanschluss (17) des vom Patienten ausgeatmeten Gases einen dritten Verlauf hat, der gegenüber dem ersten Verlauf erhöht ist, und in der zweiten Teilphase der Exspirationsphase der Druck am Patientenanschluss (17) des vom Patienten ausgeatmeten Gases einen dem ersten Verlauf entsprechenden Verlauf hat, und
während einer Inspirationsphase die Mittel zum Steuern des Gasstroms (2, 3) durch die Steuer- und Regeleinheit (7) derart angesteuert werden, dass die Ansteuerung in Inspirationsphase zwei aufeinanderfolgende Teilphasen aufweist, wobei in der ersten Teilphase der Inspirationsphase der Druck am Patientenanschluss (17) des dem Patienten zugeführten Gases einen vierten Verlauf hat, der gegenüber dem zweiten Verlauf abgesenkt ist, und in der zweiten Teilphase der Inspirationsphase der Druck am Patientenanschluss (17) des dem Patienten zugeführten Gases einen dem zweiten Verlauf entsprechenden Verlauf hat, und
wobei die Steuer- und Regeleinheit (7) ausgestaltet ist,
i) aus den von der Sensoreinheit (13, 23, 31, 31') erfassten Parametern zu bestimmen, ob an einem mit dem Patientenanschluss (17) verbundenen Patienten eine Herz-Lungen-Wiederbelebung ausgeführt wird, indem
a) die Sensoreinheit (13, 23, 31, 31') einen CO₂-Sensor (31, 31') aufweist, der ausgestaltet ist, während der Exspirationsphase den CO₂-Gehalt der von dem Patienten ausgeatmeten Luft zu bestimmen, und die Steuer- und Regeleinheit (7) ausgestaltet ist, aus dem durch den Sensor (31, 31') bestimmten CO₂-Gehalt der von dem Patienten ausgeatmeten Luft zu bestimmen, ob an dem Patienten eine Herz-Lungen-Wiederbelebung ausgeführt wird, oder indem
b) das Beatmungssystem einen Sauerstoffsättigungssensor zur Messung der Sauerstoffsättigung im Blut (SPO₂) aufweist, der mit einem Patienten verbunden werden kann, und die Steuer- und Regeleinheit (7) ausgestaltet ist, aus dem durch den Sauerstoffsättigungssensor bestimmten Wert der Sauerstoffsättigung im Blut zu bestimmen, ob an dem Patienten eine Herz-Lungen-Wiederbelebung ausgeführt wird, oder indem
c) die die Sensoreinheit (13, 23, 31, 31') einen Sauerstoffgehaltssensor aufweist, der ausgestaltet ist, während der Exspirationsphase den Sauerstoffgehalt der von dem Patienten ausgeatmeten Luft zu bestimmen, und die Steuer- und Regeleinheit (13, 23, 31, 31') ausgestaltet ist, aus dem durch den Sauerstoffgehaltssensor bestimmten Sauerstoffgehalt der von dem Patienten ausgeatmeten Luft zu bestimmen, ob an dem Patienten eine Herz-Lungen-Wiederbelebung ausgeführt wird,
ii) und, wenn dabei bestimmt worden ist, dass eine Herz-Lungen-Wiederbelebung ausgeführt wird, durch die Mittel zum Umschalten zwischen der ersten und der zweiten Betriebsart die zweite Betriebsart zu wählen.

2. Beatmungssystem nach Anspruch 1, wobei die Steuer- und Regeleinheit (7) ausgestaltet ist, während der Exspirationsphase die Mittel zum Steuern des Gasstroms (2, 3) derart anzusteuern, dass der dritte Verlauf des Drucks durch Erhöhung eines Drucksollwerts erreicht wird.

3. Beatmungssystem nach einem oder mehreren der Ansprüche 1 bis 2, wobei die Steuer- und Regeleinheit (9) ausgestaltet ist, während der Inspirationsphase die Mittel zum Steuern des Gasstroms (2, 3) derart anzusteuern, dass der vierte Verlauf des Drucks durch Verringerung eines Drucksollwerts erreicht wird.

4. Beatmungssystem nach einem der Ansprüche1, 2 oder 3, wobei die Schlauchanordnung (48, 91, 92) eine Atemgasauslassleitung (92) aufweist, die vom Patientenanschluss (17) wegführt und gegenüber dem Patientenanschluss (17) durch ein Exspirationsventil (3) geöffnet und verschlossen werden kann,
wobei das Exspirationsventil mit der Steuer- und Regeleinheit (7) verbunden ist und
wobei die Steuer- und Regeleinheit (7) ausgestaltet ist, während der Exspirationsphase das Exspirationsventil (3) derart anzusteuern, dass es gegenüber dem Beginn der Exspirationsphase zeitlich verzögert geöffnet wird.

5. Beatmungssystem nach einem oder mehreren der Ansprüche 1 bis 2 oder nach Anspruch4, wobei die Schlauchanordnung (48, 91, 92) eine Atemgaszuführleitung (91) aufweist, die von der Gasversorgungseinrichtung (9) zum Patientenanschluss (17) führt und gegenüber dem Patientenanschluss (17) durch ein Inspirationsventil (2) geöffnet und verschlossen werden kann,
wobei das Inspirationsventil (2) mit der Steuer- und Regeleinheit (7) verbunden ist,
wobei die Steuer- und Regeleinheit (7) ausgestaltet ist, während der Inspirationsphase das Inspirationsventil (2) derart anzusteuern, dass es gegenüber dem Beginn der Inspirationsphase zeitlich verzögert geöffnet wird.

6. Beatmungssystem nach einem oder mehreren der Ansprüche 1 bis5, wobei eine Umschalteinrichtung vorgesehen ist, mit der ein Benutzer die Steuer- und Regeleinheit (7) zwischen der ersten und zweiten Betriebsart umschalten kann.

7. Beatmungssystem nach Anspruch 1, wobei die Steuer- und Regeleinheit (7) ausgestaltet ist, den zeitlichen Verlauf des Drucks am Patientenanschluss während der Inspirationsphase und der Exspirationsphase zu überwachen und aus dem bei dieser Überwachung erfassten zeitlichen Verlauf des Drucks zu bestimmen, ob an dem Patienten eine Herz-Lungen-Wiederbelebung ausgeführt wird.

8. Beatmungssystem nach einem oder mehreren der Ansprüche 1 bis 7, wobei das Beatmungssystem eine Anzeigeeinheit aufweist (4), die mit der Steuer- und Regeleinheit (7) verbunden ist,
wobei die Steuer- und Regeleinheit (7) derart ausgestaltet ist, dass in der ersten Betriebsart bei Überschreiten oder Unterschreiten eines Schwellwerts durch einen von der Sensoreinheit (13, 23, 31, 31') erfassten Parameter eine erste Alarmmeldung auf der Anzeigeeinrichtung (4) ausgegeben wird, und
wobei die Steuer- und Regeleinheit (7) ausgestaltet ist, in der zweiten Betriebsart bei Überschreiten oder Unterschreiten des Schwellwerts durch den von der Sensoreinheit (13, 23, 31, 31') erfassten Parameter keine Alarmmeldung oder eine sich von der ersten Alarmmeldung unterscheidende zweite Alarmmeldung auszugeben.

9. Beatmungssystem nach Anspruch 8, wobei die Anzeigeeinheit (4) akustische Signalmittel (44) zum Ausgeben eines akustischen Alarms aufweist,
wobei die erste Alarmmeldung einen ersten akustischen Alarm umfasst und
wobei die zweite Alarmmeldung keinen akustischen Alarm oder einen sich von dem ersten unterscheidenden zweiten akustischen Alarm umfasst, dessen Lautstärke gegenüber dem ersten akustischen Alarm verringert ist.

10. Beatmungssystem nach einem oder mehreren der Ansprüche 1 bis 9, wobei eine Anzeigeeinheit (4) vorgesehen ist,
wobei die Steuer- und Regeleinheit (7) ausgestaltet ist, in der zweiten Betriebsart einen von der Sensoreinheit (13, 23, 31, 31') erfassten Parameter zu überwachen und
- bei Unterschreiten eines ersten Schwellwerts durch den Parameter eine erste Meldung durch die Anzeigeeinheit (4) auszulösen,
- bei Überschreiten des ersten Schwellwerts und Unterschreiten eines zweiten Schwellwerts, der größer als der erste Schwellwert ist, durch den Parameter eine zweite Meldung durch die Anzeigeeinheit (4) auszulösen und
- bei Überschreiten des zweiten Schwellwerts durch den Parameter eine dritte Meldung durch die Anzeigeeinheit (4) auszulösen, und
wobei sich die erste, zweite und dritte Meldung voneinander unterscheiden.

11. Beatmungssystem nach Anspruch 10, wobei die Sensoreinheit (13, 23, 31, 31') einen Sensor (31, 31') aufweist, der ausgestaltet ist, während der Exspirationsphase den CO₂-Gehalt der von dem Patienten ausgeatmeten Luft zu bestimmen, und
wobei der CO₂-Gehalt der vom Patienten ausgeatmeten Luft der überwachte Parameter ist.

12. Beatmungssystem nach einem oder mehreren der Ansprüche 1 bis 9, wobei eine Anzeigeeinheit vorgesehen ist,
wobei das Beatmungssystem einen Sensor zur Messung der Sauerstoffsättigung im Blut (SPO₂) aufweist, der mit einem Patienten verbunden werden kann,
wobei die Steuer- und Regeleinheit ausgestaltet ist, in der zweiten Betriebsart die Sauerstoffsättigung im Blut (SPO₂) zu überwachen und
- bei Unterschreiten eines ersten Schwellwerts durch die Sauerstoffsättigung im Blut (SPO₂) eine erste Meldung durch die Anzeigeeinheit auszulösen,
- bei Überschreiten des ersten Schwellwerts und Unterschreiten eines zweiten Schwellwerts, der größer als der erste Schwellwert ist, durch die Sauerstoffsättigung im Blut (SPO₂) eine zweite Meldung durch die Anzeigeeinheit auszulösen und
- bei Überschreiten des zweiten Schwellwerts durch die Sauerstoffsättigung im Blut (SPO₂) eine dritte Meldung durch die Anzeigeeinheit auszulösen, und
wobei sich die erste, zweite und dritte Meldung voneinander unterscheiden.

13. Beatmungssystem nach einem oder mehreren der Ansprüche 1 bis 12, wobei das Beatmungssystem eine Einrichtung zur selbsttätigen Durchführung einer Herz-Lungen-Wiederbelebung (1500, 1580) aufweist, die mit der Steuer- und Regeleinheit (7) verbunden ist,
wobei die Steuer- und Regeleinheit (7) ausgestaltet ist, bei einer Aktivierung der Einrichtung zur Durchführung einer Herz-Lungen-Wiederbelebung (1500, 1580) von der ersten in die zweite Betriebsart umzuschalten.

14. Beatmungssystem nach einem oder mehreren der Ansprüche 1 bis 13, wobei das Beatmungssystem einen Spannungsgenerator (1600) zum Erzeugen von Spannungspulsen aufweist, der mit der Steuer- und Regeleinheit (7) verbunden ist,
wobei der Spannungsgenerator (1600) mit Elektroden (1650) zur Verbindung mit einem Patienten versehen ist.

## Claims

1. Ventilation system comprising a gas supply device
with a hose arrangement (48, 91, 92), wherein the hose arrangement (48, 91, 92) has a patient port (17) for connection to a patient, in order to convey gas from the gas supply device (9) to the patient and in order to remove gas exhaled by the patient,
with gas flow control means (2, 3) for controlling the gas flow from the gas supply device (9) to the patient port (17) and for controlling the gas flow away from the patient port (17),
with a sensor unit (13, 23, 31, 31'), which is arranged in the hose arrangement (48, 91, 92) and is configured in such a way as to detect parameters of the gas supplied to the patient and of the gas exhaled by the patient,
with a control and regulation unit (7) for controlling the gas supply device (9) and the gas flow control means (2, 3), which control and regulation unit (7) is connected to the gas supply device (9), to the gas flow control means (2, 3) and to the sensor unit (13, 23, 31, 31'),
wherein the control and regulation unit (7) is designed such that, in a first operating mode, the gas flow control means (2, 3) are actuated by the control and regulation unit (7), during an expiration phase, in such a way that the pressure of the gas exhaled by the patient at the patient port (17) has a first profile, and the gas flow control means (2, 3) are actuated by the control and regulation unit (7), during an inspiration phase, in such a way that the pressure of the gas supplied to the patient at the patient port (17) has a second profile,
wherein the expiration phase and the inspiration phase follow each other in a continuously alternating manner,
wherein the control and regulation unit (7) is designed to have a second operating mode,
wherein the ventilation system has means for switching the control and regulation unit (7) between the first and second operating modes, and
wherein the control and regulation unit (7) is designed such that, in the second operating mode, the gas flow control means (2, 3) are actuated by the control and regulation unit (7), during the expiration phase, in such a way that the actuation in the expiration phase has two successive sub-phases, wherein, in the first sub-phase of the expiration phase, the pressure of the gas exhaled by the patient at the patient port (17) has a third profile, which is increased in relation to the first profile, and, in the second sub-phase of the expiration phase, the pressure of the gas exhaled by the patient at the patient port (17) has a profile corresponding to the first profile, and
the gas flow control means (2, 3) are actuated by the control and regulation unit (7), during an inspiration phase, in such a way that the actuation in the inspiration phase has two successive sub-phases, wherein, in the first sub-phase of the inspiration phase, the pressure of the gas supplied to the patient at the patient port (17) has a fourth profile, which is reduced in relation to the second profile, and, in the second sub-phase of the inspiration phase, the pressure of the gas supplied to the patient at the patient port (17) has a profile corresponding to the second profile, and
wherein the control and regulation unit (7) is designed
i) to determine, from the parameters detected by the sensor unit (13, 23, 31, 31'), whether cardiopulmonary resuscitation is being performed on a patient connected to the patient port (17), by the fact that
a) the sensor unit (13, 23, 31, 31') has a CO₂ sensor (31, 31') which is designed to determine, during the expiration phase, the CO₂ content of the air exhaled by the patient, and the control and regulation unit (7) is designed to determine, from the CO₂ content determined by the sensor (31, 31') in the air exhaled by the patient, whether cardiopulmonary resuscitation is being performed on the patient, or by the fact that
b) the ventilation system has an oxygen saturation sensor for measuring the oxygen saturation in the blood (SPO₂), which sensor can be connected to a patient, and the control and regulation unit (7) is designed to determine, from the blood oxygen saturation value determined by the oxygen saturation sensor, whether cardiopulmonary resuscitation is being performed on the patient, or by the fact that
c) the sensor unit (13, 23, 31, 31') has an oxygen content sensor which is designed to determine, during the expiration phase, the oxygen content of the air exhaled by the patient, and the control and regulation unit (13, 23, 31, 31') is designed to determine, from the oxygen content determined by the oxygen content sensor in the air exhaled by the patient, whether cardiopulmonary resuscitation is being performed on the patient,
ii) and, if it has thus been determined that cardiopulmonary resuscitation is being performed, to select the second operating mode by the means for switching between the first and second operating modes.

2. Ventilation system according to Claim 1, wherein the control and regulation unit (7) is designed to actuate the gas flow control means (2, 3), during the expiration phase, in such a way that the third profile of the pressure is reached by increasing a target pressure value.

3. Ventilation system according to one or both of Claims 1 and 2, wherein the control and regulation unit (9) is designed to actuate the gas flow control means (2, 3), during the inspiration phase, in such a way that the fourth profile of the pressure is reached by reducing a target pressure value.

4. Ventilation system according to one of Claims 1, 2 and 3, wherein the hose arrangement (48, 91, 92) has a respiratory gas outlet line (92), which leads away from the patient port (17) and can be opened and closed in relation to the patient port (17) by an expiration valve (3),
wherein the expiration valve is connected to the control and regulation unit (7), and
wherein the control and regulation unit (7) is designed to actuate the expiration valve (3), during the expiration phase, in such a way that it is opened with a time delay relative to the start of the expiration phase.

5. Ventilation system according to one or both of Claims 1 and 2 or according to Claim 4, wherein the hose arrangement (48, 91, 92) has a respiratory gas supply line (91), which leads from the gas supply device (9) to the patient port (17) and can be opened and closed in relation to the patient port (17) by an inspiration valve (2),
wherein the inspiration valve (2) is connected to the control and regulation unit (7),
wherein the control and regulation unit (7) is designed to actuate the inspiration valve (2), during the inspiration phase, in such a way that it is opened with a time delay relative to the start of the inspiration phase.

6. Ventilation system according to one or more of Claims 1 to 5, wherein a switching device is provided with which a user can switch the control and regulation unit (7) between the first and second operating modes.

7. Ventilation system according to Claim 1, wherein the control and regulation unit (7) is designed to monitor the time profile of the pressure at the patient port during the inspiration phase and the expiration phase and to determine, from the time profile of the pressure detected by this monitoring, whether cardiopulmonary resuscitation is being performed on the patient.

8. Ventilation system according to one of more of Claims 1 to 7, wherein the ventilation system has a display unit (4), which is connected to the control and regulation unit (7),
wherein the control and regulation unit (7) is designed such that, in the first operating mode, a first alarm message is output on the display unit (4) when a parameter detected by the sensor unit (13, 23, 31, 31') exceeds or falls below a threshold value, and
wherein the control and regulation unit (7) is designed such that, in the second operating mode, it does not output an alarm message, or it outputs a second alarm message different from the first alarm message, when the parameter detected by the sensor unit (13, 23, 31, 31') exceeds or falls below the threshold value.

9. Ventilation system according to Claim 8, wherein the display unit (4) has acoustic signalling means (44) for outputting an acoustic alarm,
wherein the first alarm message comprises a first acoustic alarm, and
wherein the second alarm message does not comprise an acoustic alarm or comprises a second acoustic alarm which is different from the first acoustic alarm and is of a reduced volume compared to that of the first acoustic alarm.

10. Ventilation system according to one or more of Claims 1 to 9, wherein a display unit (4) is provided,
wherein the control and regulation unit (7) is designed to monitor, in the second operating mode, a parameter detected by the sensor unit (13, 23, 31, 31'), and
- to trigger a first message by the display unit (4) when the parameter falls below a first threshold value,
- to trigger a second message by the display unit (4) when the parameter exceeds the first threshold value and falls below a second threshold value that is higher than the first threshold value, and
- to trigger a third message by the display unit (4) when the parameter exceeds the second threshold value, and
wherein the first, second and third messages are different from one another.

11. Ventilation system according to Claim 10, wherein the sensor unit (13, 23, 31, 31') has a sensor (31, 31') which is designed to determine, during the expiration phase, the CO₂ content of the air exhaled by the patient, and
wherein the CO₂ content of the air exhaled by the patient is the monitored parameter.

12. Ventilation system according to one or more of Claims 1 to 9, wherein a display unit is provided,
wherein the ventilation system has a sensor for measuring the oxygen saturation in the blood (SPO₂), which sensor can be connected to a patient,
wherein the control and regulation unit is designed to monitor the oxygen saturation in the blood (SPO₂) in the second operating mode, and
- to trigger a first message by the display unit when the oxygen saturation in the blood (SPO₂) falls below a first threshold value,
- to trigger a second message by the display unit when the oxygen saturation in the blood (SPO₂) exceeds the first threshold value and falls below a second threshold value that is higher than the first threshold value, and
- to trigger a third message by the display unit when the oxygen saturation in the blood (SPO₂) exceeds the second threshold value, and
wherein the first, second and third messages are different from one another.

13. Ventilation system according to one or more of Claims 1 to 12, wherein the ventilation system has a device for automatically performing cardiopulmonary resuscitation (1500, 1580), which device is connected to the control and regulation unit (7),
wherein the control and regulation unit (7) is designed to switch from the first operating mode to the second operating mode when the device for performing cardiopulmonary resuscitation (1500, 1580) is activated.

14. Ventilation system according to one or more of Claims 1 to 13, wherein the ventilation system has a voltage generator (1600) for generating voltage pulses, which voltage generator (1600) is connected to the control and regulation unit (7),
wherein the voltage generator (1600) is provided with electrodes (1650) for connection to a patient.

## Revendications

1. Système de ventilation comprenant un dispositif d'alimentation en gaz
avec un agencement de tuyaux (48, 91, 92), l'agencement de tuyaux (48, 91, 92) présentant un raccord patient (17) destiné à être relié à un patient pour conduire un gaz du dispositif d'alimentation en gaz (9) au patient et pour évacuer le gaz expiré par le patient,
avec des moyens de commande du flux de gaz (2,3) du dispositif d'alimentation en gaz (9) vers le raccord patient (17) et de commande du flux de gaz en retour depuis le raccord patient (17),
avec une unité de détection (13, 23, 31, 31') qui est disposée et arrangée dans l'agencement de tuyaux (48, 91, 92) de manière à détecter des paramètres du gaz amené au patient et expiré par le patient,
avec une unité de commande et de régulation (7) pour commander le dispositif d'alimentation en gaz (9) et les moyens de commande du flux de gaz (2, 3), qui est reliée au dispositif d'alimentation en gaz (9), aux moyens de commande du flux de gaz (2, 3) et à l'unité de détection (13, 23, 31, 31'),
dans lequel l'unité de commande et de régulation (7) est conçue de telle sorte que, dans un premier mode de fonctionnement,
pendant une phase d'expiration, les moyens de commande du flux de gaz (2, 3) sont commandés par l'unité de commande et de régulation (7) de telle sorte que la pression au raccord patient (17) du gaz expiré par le patient présente un premier profil, et que,
pendant une phase d'inspiration, les moyens de commande du flux de gaz (2, 3) sont commandés par l'unité de commande et de régulation (7) de telle sorte que la pression au raccord patient (17) du gaz amené au patient présente un deuxième profil,
dans lequel la phase d'expiration et la phase d'inspiration se succèdent continuellement en alternance,
dans lequel l'unité de commande et de régulation (7) est conçue pour avoir un deuxième mode de fonctionnement,
dans lequel des moyens sont prévus dans le système de ventilation pour commuter l'unité de commande et de régulation (7) entre le premier et le deuxième mode de fonctionnement, et
dans lequel l'unité de commande et de régulation (7) est conçue de telle sorte que, dans le deuxième mode de fonctionnement,
pendant la phase d'expiration, les moyens de commande du flux de gaz (2, 3) sont commandés par l'unité de commande et de régulation (7) de telle sorte que la commande dans la phase d'expiration présente deux phases partielles successives, la pression au raccord patient (17) du gaz expiré par le patient ayant, dans la première phase partielle de la phase d'expiration, un troisième profil qui est relevé par rapport au premier profil, et, dans la deuxième phase partielle de la phase d'expiration, la pression au raccord patient (17) du gaz expiré par le patient ayant un profil correspondant au premier profil et que,
pendant une phase d'inspiration, les moyens de commande du flux de gaz (2, 3) sont commandés par l'unité de commande et de régulation (7) de telle sorte que la commande dans la phase d'inspiration présente deux phases partielles successives, la pression au raccord patient (17) du gaz amené au patient ayant, dans la première phase partielle de la phase d'inspiration, un quatrième profil qui est abaissé par rapport au deuxième profil, et, dans la deuxième phase partielle de la phase d'inspiration, la pression au raccord patient (17) du gaz amené au patient ayant un profil correspondant au deuxième profil, et
dans lequel l'unité de commande et de régulation (7) est conçue
i) pour déterminer à partir des paramètres détectés par l'unité de détection (13, 23, 31, 31') si une réanimation cardiopulmonaire est pratiquée sur un patient relié au raccord patient (17), par le fait que
a) l'unité de détection (13, 23, 31, 31') présente un capteur de CO₂ (31, 31') qui est conçu pour déterminer la teneur en CO₂ de l'air expiré par le patient pendant la phase d'expiration, et l'unité de commande et de régulation (7) est conçue pour déterminer à partir de la teneur en CO₂ de l'air expiré par le patient, déterminée par le capteur (31, 31'), si une réanimation cardiopulmonaire est pratiquée sur le patient, ou par le fait que
b) le système de ventilation présente un capteur de saturation en oxygène pour mesurer la saturation en oxygène dans le sang (SPO₂), qui peut être relié à un patient, et l'unité de commande et de régulation (7) est conçue pour déterminer, à partir de la valeur de la saturation en oxygène du sang, déterminée par le capteur de saturation en oxygène, si une réanimation cardiopulmonaire est pratiquée sur le patient, ou par le fait que
c) l'unité de détection (13, 23, 31, 31') présente un capteur de teneur en oxygène qui est conçu pour déterminer la teneur en oxygène de l'air expiré par le patient pendant la phase d'expiration, et l'unité de commande et de régulation (13, 23, 31, 31') est conçue pour déterminer, à partir de la teneur en oxygène de l'air expiré par le patient, déterminée par le capteur de teneur en oxygène, si une réanimation cardiopulmonaire est pratiquée sur le patient,
ii) et, s'il a été déterminé qu'une réanimation cardiopulmonaire est pratiquée, pour sélectionner le deuxième mode de fonctionnement par les moyens de commutation entre le premier et le deuxième mode de fonctionnement.

2. Système de ventilation selon la revendication 1, dans lequel l'unité de commande et de régulation (7) est conçue pour commander les moyens de commande du flux de gaz (2, 3) pendant la phase d'expiration de telle sorte que le troisième profil de la pression soit obtenu en augmentant une valeur de consigne de pression.

3. Système de ventilation selon l'une ou plusieurs des revendications 1 à 2, dans lequel l'unité de commande et de régulation (9) est conçue pour commander les moyens de commande du flux de gaz (2, 3) pendant la phase d'inspiration de telle sorte que le quatrième profil de la pression soit obtenu en diminuant une valeur de consigne de pression.

4. Système de ventilation selon l'une des revendications 1, 2 ou 3, dans lequel l'agencement de tuyaux (48, 91, 92) présente une conduite de sortie de gaz respiratoire (92) qui part du raccord patient (17) et qui peut être ouverte et fermée par rapport au raccord patient (17) par une soupape d'expiration (3),
dans lequel la soupape d'expiration est reliée à l'unité de commande et de régulation (7) et
dans lequel l'unité de commande et de régulation (7) est conçue pour commander la soupape d'expiration (3) pendant la phase d'expiration de telle sorte qu'elle s'ouvre avec retard par rapport au début de la phase d'expiration.

5. Système de ventilation selon l'une ou plusieurs des revendications 1 à 2 ou selon la revendication 4, dans lequel l'agencement de tuyaux (48, 91, 92) présente une conduite d'amenée de gaz respiratoire (91) qui va du dispositif d'alimentation en gaz (9) au raccord patient (17) et qui peut être ouverte et fermée par rapport au raccord patient (17) par une soupape d'inspiration (2),
dans lequel la soupape d'inspiration (2) est reliée à l'unité de commande et de régulation (7),
dans lequel l'unité de commande et de régulation (7) est conçue pour commander la soupape d'inspiration (2) pendant la phase d'inspiration de telle sorte qu'elle s'ouvre avec retard par rapport au début de la phase d'inspiration.

6. Système de ventilation selon l'une ou plusieurs des revendications 1 à 5, dans lequel il est prévu un dispositif de commutation avec lequel un utilisateur peut commuter l'unité de commande et de régulation (7) entre le premier et le deuxième mode de fonctionnement.

7. Système de ventilation selon la revendication 1, dans lequel l'unité de commande et de régulation (7) est conçue pour surveiller l'évolution dans le temps de la pression au raccord patient pendant la phase d'inspiration et la phase d'expiration et pour déterminer, à partir de l'évolution dans le temps de la pression détectée lors de cette surveillance, si une réanimation cardiopulmonaire est pratiquée sur le patient.

8. Système de ventilation selon l'une ou plusieurs des revendications 1 à 7, dans lequel le système de ventilation présente une unité d'affichage (4) qui est reliée à l'unité de commande et de régulation (7),
dans lequel l'unité de commande et de régulation (7) est conçue de telle sorte que, dans le premier mode de fonctionnement, si un paramètre détecté par l'unité de détection (13, 23, 31, 31') devient supérieur ou inférieur à une valeur seuil, un premier message d'alarme est émis sur le dispositif d'affichage (4), et
dans lequel l'unité de commande et de régulation (7) est conçue pour, dans le deuxième mode de fonctionnement, ne pas émettre un message d'alarme ou émettre un deuxième message d'alarme différent du premier message d'alarme si le paramètre détecté par l'unité de détection (13, 23, 31, 31') devient supérieur ou inférieur à la valeur seuil.

9. Système de ventilation selon la revendication 8, dans lequel l'unité d'affichage (4) présente des moyens de signalisation acoustique (44) pour émettre une alarme acoustique,
dans lequel le premier message d'alarme comprend une première alarme acoustique et
dans lequel le deuxième message d'alarme ne comprend pas d'alarme acoustique ou comprend une deuxième alarme acoustique différente de la première alarme acoustique, dont l'intensité sonore est diminuée par rapport à la première alarme acoustique.

10. Système de ventilation selon l'une ou plusieurs des revendications 1 à 9, dans lequel il est prévu une unité d'affichage (4),
dans lequel l'unité de commande et de régulation (7) est conçue pour, dans le deuxième mode de fonctionnement, surveiller un paramètre détecté par l'unité de détection (13, 23, 31, 31') et
- si le paramètre devient inférieur à une première valeur seuil, pour déclencher un premier message par l'unité d'affichage (4),
- si le paramètre devient supérieur à la première valeur seuil et inférieur à une deuxième valeur seuil, qui est supérieure à la première valeur seuil, pour déclencher un deuxième message par l'unité d'affichage (4) et
- si le paramètre devient supérieur à la deuxième valeur seuil, pour déclencher un troisième message par l'unité d'affichage (4), et
dans lequel les premier, deuxième et troisième messages sont différents les uns des autres.

11. Système de ventilation selon la revendication 10, dans lequel l'unité de détection (13, 23, 31, 31') présente un capteur (31, 31') qui est conçu pour déterminer la teneur en CO₂ de l'air expiré par le patient pendant la phase d'expiration, et
dans lequel la teneur en CO₂ de l'air expiré par le patient est le paramètre surveillé.

12. Système de ventilation selon l'une ou plusieurs des revendications 1 à 9, dans lequel il est prévu une unité d'affichage,
dans lequel le système de ventilation présente un capteur pour mesurer la saturation en oxygène dans le sang (SPO₂) qui peut être relié à un patient,
dans lequel l'unité de commande et de régulation est conçue pour, dans le deuxième mode de fonctionnement, surveiller la saturation en oxygène dans le sang (SPO₂) et
- si la saturation en oxygène dans le sang (SPO₂) devient inférieure à une première valeur seuil, pour déclencher un premier message par l'unité d'affichage,
- si la saturation en oxygène dans le sang (SPO₂) devient supérieure à la première valeur seuil et inférieure à une deuxième valeur seuil, qui est supérieure à la première valeur seuil, pour déclencher un deuxième message par l'unité d'affichage et
- si la saturation en oxygène dans le sang (SPO₂) devient supérieure à la deuxième valeur seuil, pour déclencher un troisième message par l'unité d'affichage, et
dans lequel les premier, deuxième et troisième messages sont différents les uns des autres.

13. Système de ventilation selon l'une ou plusieurs des revendications 1 à 12, dans lequel le système de ventilation présente un dispositif pour pratiquer automatiquement une réanimation cardiopulmonaire (1500, 1580), qui est relié à l'unité de commande et de régulation (7),
dans lequel l'unité de commande et de régulation (7) est conçue pour effectuer une commutation du premier au deuxième mode de fonctionnement en cas d'activation du dispositif pour pratiquer une réanimation cardiopulmonaire (1500, 1580).

14. Système de ventilation selon l'une ou plusieurs des revendications 1 à 13, dans lequel le système de ventilation comprend un générateur de tension (1600) pour générer des impulsions de tension, qui est relié à l'unité de commande et de régulation (7),
dans lequel le générateur de tension (1600) est muni d'électrodes (1650) pour la liaison à un patient.
